# EUROPÄISCHE PATENTSCHRIFT

(11) **EP 2 340 784 B1**
(45) Veröffentlichungstag und Bekanntmachung des Hinweises auf die Patenterteilung: **23.12.2015**
(21) Anmeldenummer: 10197317.0
(22) Anmeldetag: 29.12.2010
(51) Int. Cl.: A61F 2/00, D04B 21/12

(54) **Textiles Netz, insbesondere zur Prolapsversorgung bzw. -behandlung oder zur Vermeidung von Dyspareunia**
Textile net, in particular for prolapse treatment and care for or preventing dyspareunia
Réseau textile, notamment pour l'alimentation ou le traitement de prolapsus ou pour éviter une dyspareunie

(30) Priorität: 31.12.2009 DE 102009060799
(43) Veröffentlichungstag der Anmeldung: 06.07.2011
(73) Patentinhaber: Aesculap AG, 78532 Tuttlingen/Donau (DE); ITV Denkendorf Produktservice GmbH, 73770 Denkendorf (DE)
(72) Erfinder: Odermatt, Erich, 8200, Schaffhausen (CH); Weis, Christine, 8190, Sant Cugat del Vallés (ES); Müller, Erhard, 70565, Stuttgart (DE); Schmees, Hans-Gerd, 72827, Wannweil (DE)
(74) Vertreter: Patentanwälte Ruff, Wilhelm, Beier, Dauster & Partner mbB

(56) Entgegenhaltungen:
- WO-A2-2008/058163
- DE-A1- 10 019 604
- DE-A1- 10 041 347
- DE-A1- 19 912 648
- DE-A1-102004 025 404
- DE-A1-102004 051 487
- US-A- 5 686 090
- US-A1- 2003 114 866
- US-A1- 2005 070 829
- US-A1- 2008 208 360
- US-A1- 2009 024 147
- US-B2- 7 628 155

## Beschreibung

Die vorliegende Erfindung betrifft ein textiles Netz, ein chirurgisches Implantat sowie ein chirurgisches Kit zur Versorgung bzw. Behandlung von Prolapsen oder zur Vermeidung von Dyspareunia.

Als Prolaps wird in der Medizin der unnatürliche Vorfall eines Organs oder eines Organteils durch eine natürliche oder pathologisch bedingte Körperöffnung bezeichnet. Das Auftreten von Prolapsen ist dabei in den allermeisten Fällen auf ein Versagen der haltenden Bänder zurückzuführen. Ein häufig auftretender Prolaps ist der sogenannte Uterusprolaps. Hierunter versteht man einen Gebärmuttervorfall, ein Durchdrücken der Gebärmutter durch den Geburtskanal hindurch, zum Teil bis zum Austritt innerhalb der nun ausgestülpten Vagina außerhalb des Körpers (Prolapsus uteri et vaginae). Der Uterusprolaps stellt dabei die extreme Form einer Gebärmuttersenkung (descensus uteri) dar.

Zur erfolgreichen Behandlung eines Prolapses reicht es in der Regel nicht aus, die erschlafften oder gegebenenfalls abgerissenen Bänder zu ersetzen, da gewöhnlich auch das umliegende Bindegewebe relaxiert ist. Aus diesem Grund kommt nur eine flächige Unterstützung der hervorgetretenen Organe bzw. Organteile in Frage. Üblicherweise werden hierfür Netze eingesetzt. Die verwendeten Netze haben jedoch den Nachteil, dass sie häufig zu Mobilitätseinschränkungen bei Patienten führen. Beispielsweise können Prolapsnetze die Ursache für Schmerzempfindungen beim Geschlechtsverkehr (Dyspareunia) sein. Außerdem kann es zu unerwünschten Gewebeerosionen unterhalb der zu stützenden Organe bzw. Organteile kommen, wodurch wiederum der Heilungsverlauf beeinträchtigt wird. Hinzu kommt, dass sich eine feste und sichere Aufhängung der Netze in einer Prolapszone in vielen Fällen als schwierig erweist, woraus ein generell erhöhtes Risiko für ein Prolapsrezidiv resultiert.

WO 2008/058163 A2 beschreibt ein Netz mit relativ weichem, flexiblem Körper und relativ starken Armen, die vom Körper abgehen.

Die vorliegende Erfindung stellt sich daher die Aufgabe, ein textiles Netz zur Versorgung bzw. Behandlung von Prolapsen bereitzustellen, welches aus dem Stand der Technik bekannte Unzulänglichkeiten vermeidet, insbesondere das Auftreten von Gewebeerosionen reduziert oder weitgehend verhindert, zu keiner nennenswerten Einschränkung der Beweglichkeit eines Patienten führt und insbesondere das Risiko einer erneuten Prolapsmanifestation minimiert.

Diese Aufgabe wird erfindungsgemäß gelöst durch ein textiles Netz mit den Merkmalen des unabhängigen Anspruchs 1. Bevorzugte Ausführungsformen des textilen Netzes sind Gegenstand der abhängigen Ansprüche 2 bis 12. Ein weiterer Aspekt der Erfindung betrifft chirurgische Implantate mit den Merkmalen des unabhängigen Ansprüchs 13. Bevorzugte Ausführungsformen der chirurgischen Implantate sind in den Ansprüchen 14 und 15 wiedergegeben. Der Wortlaut sämtlicher Ansprüche wird hiermit durch Bezugnahme zum Inhalt der vorliegenden Beschreibung gemacht.

Bei dem erfindungsgemäßen Netz handelt es sich um ein textiles Netz, insbesondere zur Anwendung bei der Beckenbodenrekonstruktion, vorzugsweise zur Anwendung bei der Versorgung oder Behandlung von Prolapsen oder prolapsartigen Erkrankungen, oder der Vermeidung von Dyspareunia. Das Netz weist einen mittigen Netzabschnitt und mindestens zwei, insbesondere zwei, seitliche Netzabschnitte beidseits des mittigen Netzabschnittes auf. Das Netz ist vorzugsweise zumindest teilweise, insbesondere nur teilweise, anisotrop elastisch ausgebildet. Der mittige Netzabschnitt ist nicht resorbierbar, ausgebildet. Die mindestens zwei seitlichen Netzabschnitte sind nicht resorbierbar ausgebildet.

Das erfindungsgemäße Netz bietet gegenüber konventionellen Netzen, wie im Folgenden noch eingehender ausgeführt wird, deutliche Vorteile bei der Beckenbodenrekonstruktion, insbesondere bei der Versorgung bzw. Behandlung von Prolapsen bzw. bei der Vermeidung von Dyspareunia.

In einer beispielhaften Ausführungsform sind der mittige Netzabschnitt nicht resorbierbar und die beiden seitlichen Netzabschnitte resorbierbar ausgebildet. Durch die nicht resorbierbare Ausbildung des mittigen Netzabschnittes ist beispielsweise eine dauerhafte Unterstützung von prolapierten Organen bzw. Organteilen möglich. Wie bereits erwähnt, sind die beiden seitlichen Netzabschnitte vorzugsweise zur Aufhängung des Netzes in einer menschlichen oder tierischen Körperdefektzone, insbesondere Prolapszone, vorgesehen. Die resorbierbare Ausbildung der seitlichen Netzabschnitte hat den Vorteil, dass bei dieser Ausführungsform im Endeffekt nur der mittige Netzabschnitt als Fremdmaterial im Körper eines Patienten verbleibt. Auf diese Weise kann somit die Menge an Fremdmaterial im Körper eines Patienten verringert werden, wodurch sich die Lebensqualität des Patienten deutlich verbessern lässt. Hinzu kommt, dass die Resorption von Materialien im Körper eines Patienten in der Regel von entzündlichen Reaktionen begleitet ist, welche die Bildung von Narbengewebe stimulieren. Das gebildete Narbengewebe wiederum kann in besonders vorteilhafter Weise die Aufhängungsfunktion der beiden seitlichen Netzabschnitte nach deren Resorption übernehmen.

In einer möglichen alternativen Ausführungsform sind der mittige Netzabschnitt resorbierbar und zumindest einer der beiden seitlichen Netzabschnitte, insbesondere beide Netzabschnitte, nicht resorbierbar oder teilresorbierbar ausgebildet.

Zur Herstellung des Netzes, insbesondere des mittigen Netzabschnittes und/oder zumindest eines der beiden seitlichen Netzabschnitte, vorzugsweise von beiden seitlichen Netzabschnitten, können grundsätzlich alle bioverträglichen Materialien, insbesondere Polymere, verwendet werden. Unter einem bioverträglichen Material soll im Sinne der vorliegenden Erfindung ein körper- bzw. gewebeverträgliches Material verstanden werden. Dabei können der mittige Netzabschnitt und die beiden seitlichen Netzabschnitte grundsätzlich aus dem gleichen Material oder aus verschiedenen Materialien gebildet sein. Bevorzugt sind die beiden seitlichen Netzabschnitte aus dem gleichen Material und der mittige Netzabschnitt aus einem anderen Material gebildet. Die Materialien können resorbierbar, teilresorbierbar oder nicht resorbierbar sein. Besonders bevorzugt ist es, wenn der mittige Netzabschnitt aus einem nicht resorbierbaren, gegebenenfalls aus einem teilresorbierbaren, Material und die beiden seitlichen Netzabschnitte aus einem resorbierbaren Material gebildet sind.

Der mittige Netzabschnitt und/oder zumindest einer der beiden, insbesondere die beiden, seitlichen Netzabschnitte, besonders bevorzugt nur die beiden seitlichen Netzabschnitte, sind in einer weitergehenden Ausführungsform aus Polyhydroxyalkanoaten bzw. Copolymeren davon gebildet, welche vorzugsweise ausgewählt sind aus der Gruppe bestehend aus Polyglykolid, Polylaktid, Poly-ε-caprolacton, Polytrimethylencarbonat, Poly-para-dioxanon, Poly-3-hydroxybutyrat, Poly-4-hydroxybutyrat, Copolymere davon und Blends davon. Ein bevorzugtes Copolymer ist ein Copolymer auf Basis von Glykolid und Laktid.

Der mittige Netzabschnitt und/oder zumindest einer der beiden, insbesondere die beiden, seitlichen Netzabschnitte, besonders bevorzugt nur die beiden seitlichen Netzabschnitte, bzw. Fäden des mittigen Netzabschnittes und/oder zumindest eines der beiden, insbesondere der beiden, seitlichen Netzabschnitte, besonders bevorzugt nur der beiden seitlichen Netzabschnitte, sind in einer weiteren Ausführungsform aus einem biologischen Material, insbesondere Biopolymer (natürlich vorkommendes Polymer) und/oder einem davon abgeleiten Polymer gebildet. Bei dem biologischen Material handelt es sich vorzugsweise um Proteine, insbesondere extrazelluläre und/oder faserförmige Proteine, Polysaccharide, insbesondere Mucopolysaccharide und/oder Cellulosederivate wie beispielsweise Alkylcellulosen, und/oder um biologisches, vorzugsweise tierisches, Gewebe. Das Gewebe ist in der Regel xenogenen, insbesondere porcinen, bovinen oder equinen, Ursprungs. Bevorzugt ist das biologische Material aus der Gruppe bestehend aus Gelatine, Collagen, Retikulin, Elastin, Fibronektin, Albumin, Methylcellulose, Hydroxymethylcellulose, Hydroxyethylcellulose, Hydroxypropylcellulose, Hydroxybutylcellulose, Hydroxyethylmethylcellulose, Hydroxypropylmethylcellulose, Carboxymethylcellulose, Chitosan, Dermatansulfat, Keratansulfat, Chondroitinsulfat, Hyaluronsäure, Stärke, Amylopektin, Amylose, Dextran, Polyvinylalkohol, Salze davon, Pericard und Kombinationen davon ausgewählt.

In einer weiteren Ausführungsform sind der mittige Netzabschnitt und/ oder zumindest einer der beiden, insbesondere die beiden, seitlichen Netzabschnitte, besonders bevorzugt nur der mittige Netzabschnitt, bzw. Fäden des mittigen Netzabschnittes und/oder zumindest eines der beiden, insbesondere der beiden, seitlichen Netzabschnitte, besonders bevorzugt nur des mittigen Netzabschnittes, aus einem nicht resorbierbaren Polymer bzw. Copolymer gebildet, welches vorzugsweise aus der Gruppe bestehend aus Polyolefine, insbesondere Polyethylen, beispielsweise Polyethylen hoher Dichte (HDPE), Polyethylen niedriger Dichte (LDPE), hochmolekulares Polyethylen (HMWPE) und/oder ultra-hochmolekulares Polyethylen (UHMWPE), Polypropylen, Polytetrafluorethylen und/oder Polyhexafluoropropylen, Polyester, insbesondere Polyethylenterephthalat (PET) und/oder Polybutylenterephthalat (PBT), Polyamide, Polyurethane, Copolymere davon und Kombinationen, insbesondere Blends, davon ausgewählt ist. Unter dem Begriff Copolymer im Sinne der vorliegenden Erfindung soll ein Polymer verstanden werden, welches aus zumindest zwei unterschiedlichen Monomereinheiten zusammengesetzt ist. Entsprechend kann es sich bei einem Copolymer im Sinne der vorliegenden Erfindung beispielsweise um ein Terpolymer handeln.

Erfindungsgemäß kann es weiterhin vorgesehen sein, dass das Netz, insbesondere der mittige Netzabschnitt und/oder zumindest einer der beiden seitlichen Netzabschnitte, insbesondere beide seitlichen Netzabschnitte, auch aus Kombinationen von Materialien, insbesondere Polymeren bzw. Biopolymeren und/oder biologischen Geweben, wie sie in den vorhergehenden Ausführungsformen aufgeführt wurden, gebildet ist.

Die Netzabschnitte können grundsätzlich entlang ihrer Längs- oder Querränder miteinander verbunden sein. Bevorzugt sind der mittige Netzabschnitt und die beiden seitlichen Netzabschnitte aber entlang ihrer Längsränder miteinander verbunden.

In einer möglichen Ausführungsform weist das Netz zumindest zwei, insbesondere zwei, zusätzliche seitliche Netzabschnitte auf. Mit anderen Worten kann das erfindungsgemäße Netz auf einem mittigen Netzabschnitt und vier seitlichen Netzabschnitten basieren. Zwei der seitlichen Netzabschnitte sind vorzugsweise entlang ihrer Längsränder mit dem Längsrand des mittigen Netzabschnittes verbunden. Die zwei anderen seitlichen Netzabschnitte sind bevorzugt entlang ihrer Querränder mit dem Querrand des mittigen Netzabschnittes verbunden.

In einer bevorzugten Ausführungsform ist das Netz, insbesondere der mittige Netzabschnitt und/oder zumindest einer der beiden seitlichen Netzabschnitte, vorzugsweise die beiden seitlichen Netzabschnitte, streifenförmig ausgebildet. Besonders bevorzugt weist das Netz in toto, d.h. einschließlich des mittigen Netzabschnittes und der beiden seitlichen Netzabschnitte, einen streifenförmigen Aufbau auf.

In einer besonders bevorzugten Ausführungsform sind der mittige Netzabschnitt und die beiden seitlichen Netzabschnitte jeweils streifenförmig ausgebildet und die Netzabschnitte entlang ihrer Längsränder miteinander verbunden.

Das Netz, insbesondere der mittige Netzabschnitt und/oder zumindest einer der beiden seitlichen Netzabschnitte, insbesondere beide seitlichen Netzabschnitte, kann grundsätzlich zwei-, drei- oder mehrlagig aufgebaut sein. Bevorzugt ist das Netz, insbesondere der mittige Netzabschnitt und/oder zumindest einer der beiden seitlichen Netzabschnitte, vorzugsweise die beiden seitlichen Netzabschnitte, einlagig ausgebildet. Durch eine einlagige Ausbildung des Netzes kann mit besonderem Vorteil eine gewisse Grundflexibilität erzeugt werden.

In einer weitergehenden Ausführungsform ist der Mittelabschnitt zweilagig aufgebaut, wobei eine Lage aus einem resorbierbaren Material und die andere Lage aus einem nicht resorbierbaren Material gebildet ist. Die Resorption der einen Lage induziert die Bildung von Binde- oder Narbengewebe, welches mit besonderem Vorteil eine zusätzliche Stützung oder Anhebung von prolapierten Organen bzw. Organteilen bewirkt. Weiterhin kann eine Lage, vorzugsweise die aus resorbierbarem Material gebildete Lage, als Folie, insbesondere als Schutzfolie zur Vermeidung von Gewebeerosionen, ausgebildet sein.

Der mittige Netzabschnitt kann eine Einzelbreite besitzen, die im Wesentlichen 1/7 der Gesamtbreite des Netzes entspricht. Die beiden seitlichen Netzabschnitte können jeweils eine Einzelbreite besitzen, die im Wesentlichen 3/7 der Gesamtbreite des Netzes entspricht.

In einer weiteren bevorzugten Ausführungsform ist der mittige Netzabschnitt zur Stützung von aus einer natürlichen oder pathologisch bedingten Körperöffnung hervorgetretenen Organen oder Organteilen vorgesehen. Die beiden seitlichen Netzabschnitte sind vorzugsweise zur Aufhängung des Netzes in einer menschlichen oder tierischen Körperdefektzone, vorzugsweise Prolapszone, vorgesehen.

Die im Folgenden beschriebenen Angaben in Längs- und/oder Querrichtung des Netzes, insbesondere des mittigen Netzabschnittes und/oder der beiden seitlichen Netzabschnitte, beziehen sich vorzugsweise auf Angaben parallel zur textilen Bindungsrichtung, vorzugsweise Wirkrichtung, (Angaben in Längsrichtung) und auf Angaben quer zur textilen Bindungsrichtung, vorzugsweise Wirkrichtung, (Angaben in Querrichtung) des Netzes, insbesondere des mittigen Netzabschnittes und/oder der beiden seitlichen Netzabschnitte.

Wie bereits erwähnt, ist es bevorzugt, wenn das erfindungsgemäße Netz zumindest teilweise eine von der Richtung abhängige Elastizität besitzt. Vorzugsweise besitzt das Netz in Längsrichtung des mittigen Netzabschnittes und/oder in Längsrichtung zumindest einer der beiden seitlichen Netzabschnitte, vorzugsweise der beiden seitlichen Netzabschnitte, eine andere Elastizität als in Querrichtung davon.

In einer weitergehenden Ausführungsform ist das Netz zumindest im Bereich des mittigen Netzabschnittes anisotrop elastisch ausgebildet. Grundsätzlich ist es möglich, dass das Netz sowohl im Bereich des mittigen Netzabschnittes als auch im Bereich von zumindest einem seitlichen Netzabschnitt, insbesondere in den Bereichen der beiden seitlichen Netzabschnitte, anisotrop elastisch ausgebildet ist. Erfindungsgemäß ist es jedoch bevorzugt, wenn das Netz nur im Bereich des mittigen Netzabschnittes anisotrop elastisch ausgebildet ist.

In einer besonders bevorzugten Ausführungsform ist das Netz in Querrichtung des mittigen Netzabschnittes elastischer ausgebildet als in Längsrichtung des mittigen Netzabschnittes. Der mittige Netzabschnitt kann dabei in Längsrichtung eine Elastizität aufweisen, die im Wesentlichen der Elastizität der seitlichen Netzabschnitte entspricht, insbesondere in deren Längs- und/oder Querrichtung. Bevorzugt weist das Netz in Querrichtung des mittigen Netzabschnittes eine Dehnbarkeit zwischen 200 und 250 %, insbesondere 210 und 240 %, vorzugsweise 220 und 230 %, auf. In Längsrichtung des mittigen Netzabschnittes weist das Netz vorzugsweise eine Dehnbarkeit zwischen 100 und 170 %, insbesondere 110 und 160 %, vorzugsweise 115 und 155 %, auf.

In einer weitergehenden Ausführungsform ist das Netz im Bereich des mittigen Netzabschnittes elastischer ausgebildet als in den Bereichen der beiden seitlichen Netzabschnitte. Dies hat den Vorteil, dass sich der mittige Netzabschnitt besser an Veränderungen, insbesondere Volumen- und/oder Bewegungsänderungen, von zu stützenden Organen bzw. Organteilen, beispielsweise Blasenfüllungen, Rektumsbewegungen und dergleichen, anpassen kann. Umgekehrt ermöglicht eine weniger elastische Ausbildung der beiden seitlichen Netzabschnitte eine festere bzw. straffere und damit sichere Aufhängung des Netzes, beispielsweise im Bereich einer Prolapszone, wodurch das Risiko eines erneuten Absinkens von gestützten Organen bzw. Organteilen und damit die Manifestation eines erneuten Prolapses deutlich reduziert werden kann. Entsprechende Ausführungen gelten sinngemäß auch für das im Folgenden noch näher beschriebene chirurgische Implantat, das ebenfalls Gegen-stand der vorliegenden Erfindung ist.

Vorzugsweise ist das Netz in Querrichtung des mittigen Netzabschnittes elastischer ausgebildet als in Längs- und/oder Querrichtung, vorzugsweise Längs- und Querrichtung, zumindest einer der beiden seitlichen Netzabschnitte, vorzugsweise der beiden seitlichen Netzabschnitte. Das Netz besitzt in einer weiteren Ausführungsform in den Bereichen der beiden seitlichen Netzabschnitte im Wesentlichen die gleiche Elastizität. Insbesondere kann das Netz in den Bereichen der beiden seitlichen Netzabschnitte im Wesentlichen isotrop elastisch ausgebildet sein. Erfindungsgemäß kann es insbesondere vorgesehen sein, dass die beiden seitlichen Netzabschnitte in Längs- und Querrichtung die gleiche Elastizität besitzen. Bevorzugt weist das Netz in Längs- und/oder Querrichtung, vorzugsweise Längs- und Querrichtung, zumindest eines der beiden seitlichen Netzabschnitte, insbesondere der beiden seitlichen Netzabschnitte, eine Dehnbarkeit zwischen 100 und 170 %, insbesondere 110 und 160 %, vorzugsweise 115 und 155 %, auf. Grundsätzlich kann das Netz im Bereich seiner beiden seitlichen Netzabschnitte aber auch unterschiedliche Elastizitäten aufweisen. Dies kann abhängig von den besonderen anatomischen Gegebenheiten der Implantationsstelle von Vorteil sein.

Eine anisotrop elastische Ausbildung bzw. anisotrope Elastizität des Netzes, insbesondere des mittigen Netzabschnittes und/oder zumindest eines der beiden seitlichen Netzabschnitte, vorzugsweise der beiden seitlichen Netzabschnitte, ist vorzugsweise durch die textile Struktur, insbesondere die textilen Eigenschaften, des Netzes, insbesondere des mittigen Netzabschnittes und/oder zumindest eines der beiden seitlichen Netzabschnitte, vorzugsweise der beiden seitlichen Netzabschnitte, bedingt. Geeignete Parameter, welche zur Ausbildung einer anisotropen Elastizität herangezogen werden können, sind beispielsweise Fadenmaterial, Fadentiter, Fadendurchmesser, Netzöffnungsweiten, Netzöffnungsdichten, Flächengewicht oder Kombinationen davon.

Gemäß einer besonders bevorzugten Ausführungsform ist das Netz als sogenanntes Gradientennetz ausgebildet. Das Gradientennetz ist dabei vorzugsweise einstückig oder monolithisch gefertigt. Unter einem Gradientenetz im Sinne der vorliegenden Erfindung soll ein Verbundnetz oder eine Art Verbundnetz aus zwei oder mehr, vorzugsweise zwei oder drei, verschiedenen Einzelnetzen, welche vorzugsweise flächig nebeneinander angeordnet und miteinander verbunden sind, verstanden werden. Hierdurch lassen sich in besonders vorteilhafter Weise unterschiedliche textile Eigenschaften in einem Netz verwirklichen (Ausbildung eines sogenannten polyfunktionalen Netzes). Bevorzugt weist der mittige Netzabschnitt eine andere textile Netzstruktur bzw. einen anderen textilen Aufbau, insbesondere andere textile Eigenschaften, auf als die beiden seitlichen Netzabschnitte. Die beiden seitlichen Netzabschnitte besitzen vorzugsweise die gleiche textile Netzstruktur bzw. den gleichen textilen Aufbau, insbesondere die gleichen textilen Eigenschaften. Grundsätzlich können die beiden seitlichen Netzabschnitte auch unterschiedliche textile Netzstrukturen besitzen. Erfindungsgemäß kann es insbesondere vorgesehen sein, dass jeder Netzabschnitt des Netzes eine andere textile Netzstruktur besitzt und/oder aus einem anderen Netzmaterial gebildet ist.

Das Netz, insbesondere der mittige Netzabschnitt und/oder zumindest einer der beiden seitlichen Netzabschnitte, vorzugsweise die beiden seitlichen Netzabschnitte, besitzt in einer weiteren geeigneten Ausführungsform ein anisotropes Reißkraftverhalten. Bevorzugt weist das Netz in Längsrichtung des mittigen Netzabschnittes und/oder zumindest eines der beiden seitlichen Netzabschnitte, vorzugsweise der beiden seitlichen Netzabschnitte, eine andere Reißkraft auf als in Querrichtung davon. Besonders bevorzugt ist die Reißkraft des Netzes, insbesondere des mittigen Netzabschnittes und/oder zumindest eines der beiden seitlichen Netzabschnitte, vorzugsweise der beiden seitlichen Netzabschnitte, in Längsrichtung höher als in Querrichtung.

Besonders bevorzugt weist das Netz im Bereich des mittigen Netzabschnittes eine kleinere Reißkraft auf als in den Bereichen der beiden seitlichen Netzabschnitte. Insbesondere kann die Reißkraft in Längs- und/oder Querrichtung, vorzugsweise in Längs- und Querrichtung, des mittigen Netzabschnittes, kleiner sein als die Reißkraft in Längs- und/ oder Querrichtung, vorzugsweise in Längs- und Querrichtung, der beiden seitlichen Netzabschnitte. Die beiden seitlichen Netzabschnitte weisen vorzugsweise im Wesentlichen das gleiche Reißkraftverhalten, insbesondere in Längs- und/oder Querrichtung, auf. Beispielsweise kann die Reißkraft in Längsrichtung des mittigen Netzabschnittes zwischen 40 und 60 N/cm, insbesondere 45 und 55 N/cm, liegen. In Querrichtung kann der mittige Netzabschnitt eine Reißkraft zwischen 20 und 35 N/cm, insbesondere 25 und 30 N/cm, aufweisen. Die beiden seitlichen Netzabschnitte können in Längsrichtung beispielsweise eine Reißkraft zwischen 50 und 70 N/cm, insbesondere 55 und 65 N/cm, aufweisen. In Querrichtung können die beiden seitlichen Netzstreifen eine Reißkraft zwischen 30 und 50 N/cm, insbesondere 35 und 45 N/cm, aufweisen.

In einer weiteren Ausführungsform weist das Netz Fäden mit unterschiedlichem Titer auf. Bevorzugt weisen Fäden des mittigen Netzabschnittes einen anderen, vorzugsweise feineren bzw. kleineren, Titer (längenbezogene Masse) auf als Fäden der beiden seitlichen Netzabschnitte. Erfindungsgemäß kann es weiterhin vorgesehen sein, dass die beiden seitlichen Netzabschnitte Fäden mit unterschiedlichem Titer besitzen. Bevorzugt weisen die beiden seitlichen Netzabschnitte jeweils Fäden mit gleichem Titer auf. Typischerweise haben Fäden des mittigen Netzabschnittes einen Titer zwischen 50 und 120 dtex, insbesondere 70 und 110 dtex, vorzugsweise 80 und 100 dtex. Fäden der beiden seitlichen Netzabschnitte können beispielsweise einen Titer zwischen 120 und 180 dtex, insbesondere 130 und 170 dtex, vorzugsweise 140 und 160 dtex, besitzen.

In einer weiteren vorteilhaften Ausführungsform weist das Netz Fäden mit unterschiedlichem Fadendurchmesser auf. Bevorzugt weisen Fäden des mittigen Netzabschnittes einen anderen, besonders bevorzugt kleineren, Durchmesser auf als Fäden der beiden seitlichen Netzabschnitte. Grundsätzlich können die beiden seitlichen Netzabschnitte auch Fäden mit unterschiedlichem Durchmesser besitzen. Bevorzugt besitzen die beiden seitlichen Netzabschnitte jeweils Fäden mit gleichem Durchmesser. Zum Beispiel können Fäden des mittigen Netzabschnittes einen Durchmesser zwischen 50 µm und 125 µm, insbesondere 70 und 120 µm, vorzugsweise 90 und 110 µm, besitzen. Die beiden seitlichen Netzabschnitte besitzen beispielsweise Fäden mit einem Durchmesser zwischen 130 und 170 µm, insbesondere 135 und 160 µm, vorzugsweise 140 und 150 µm.

In einer weiteren vorteilhaften Ausführungsform weist das Netz Bereiche mit unterschiedlich geformten Netzöffnungen auf. Die Netzöffnungen können grundsätzlich kreisförmig, oval, ellipsoid und/oder polygon ausgebildet sein. Bevorzugt sind polygone Netzöffnungen. Beispiele für polygon ausgebildete Netzöffnungen sind viereckig, fünfeckig (pentagonal) und/oder sechseckig (hexagonal) bzw. wabenförmig ausgebildete Netzöffnungen. Beispiele für mögliche viereckige Netzöffnungen sind quadratische, rechteckige, rautenförmige, parallelogrammförmige und/oder trapezförmige Netzöffnungen. Die beiden seitlichen Netzabschnitte besitzen jeweils vorzugsweise gleich geformte Netzöffnungen. Zum Beispiel können die beiden seitlichen Netzabschnitte hexagonale bzw. wabenförmige Netzöffnungen besitzen. Die Netzöffnungen können als Maschen oder Poren ausgebildet sein. In der Regel sind die Netzöffnungen als Maschen ausgebildet. Grundsätzlich können die beiden seitlichen Netzabschnitte auch unterschiedlich geformte Netzöffnungen aufweisen.

In einer weiteren bevorzugten Ausführungsform weist das Netz Öffnungen mit unterschiedlichen lichten Weiten auf. Bevorzugt weisen Öffnungen des mittigen Netzabschnittes eine andere, insbesondere kleinere, lichte Weite auf als Öffnungen der beiden seitlichen Netzabschnitte. Grundsätzlich können die beiden seitlichen Netzabschnitte aber auch Öffnungen mit unterschiedlichen lichten Weiten besitzen. Vorzugsweise besitzen die beiden seitlichen Netzabschnitte Öffnungen mit gleichen lichten Weiten. Der mittige Netzabschnitt besitzt vorzugsweise Öffnungen mit einer lichten Weite zwischen 0,5 und 1,5 mm, insbesondere von ca. 1 mm. Die beiden seitlichen Netzabschnitte können Öffnungen mit einer lichten Weite zwischen 2 und 4 mm, insbesondere 2,5 und 3,8 mm, besonders bevorzugt 2,8 und 3,6 mm, besitzen. Unter dem Begriff "lichte Weite" soll im Sinne der vorliegenden Erfindung der Innendurchmesser von Öffnungen des Netzes, insbesondere des mittigen Netzabschnittes und/oder zumindest eines der beiden seitlichen Netzabschnitte, vorzugsweise der beiden seitlichen Netzabschnitte, verstanden werden. Abhängig davon, wie die Öffnungen ausgebildet sind, kann der Begriff "lichte Weite" mit lichter Maschen- oder Porenweite, vorzugsweise lichter Maschenweite, gleich zu setzen sein.

In einer weiteren vorteilhaften Ausführungsform weist das Netz Bereiche mit unterschiedlichen Öffnungsdichten auf. Bevorzugt weist der mittige Netzabschnitt eine andere, vorzugsweise höhere, Öffnungsdichte auf als die beiden seitlichen Netzabschnitte. Grundsätzlich können die beiden seitlichen Netzabschnitte unterschiedliche Öffnungsdichten aufweisen. Erfindungsgemäß ist es jedoch bevorzugt, wenn die beiden seitlichen Netzabschnitte jeweils gleiche Öffnungsdichten besitzen. Beispielsweisweise kann der mittige Netzabschnitt eine Öffnungsdichte zwischen 80 und 135 Öffnungen pro cm², insbesondere 90 und 125 Öffnungen pro cm², bevorzugt 100 und 120 Öffnungen pro cm², aufweisen. Die beiden seitlichen Netzabschnitte besitzen vorzugsweise eine Öffnungsdichte zwischen 40 und 75 Öffnungen pro cm², insbesondere 50 und 70 Öffnungen pro cm², bevorzugt 55 und 65 Öffnungen, pro cm². Abhängig von der konkreten Ausbildung der Öffnungen, beispielsweise als Maschen oder Poren, kann mit dem Begriff "Öffnungsdichte" die Maschen- oder Porendichte, vorzugsweise Maschendichte, gemeint sein.

Erfindungsgemäß ist es weiterhin bevorzugt, dass der mittige Netzabschnitt eine andere Maschenstruktur, insbesondere eine kleinere lichte Maschenweite und/oder höhere Maschendichte, besitzt als die beiden seitlichen Netzabschnitte. Erfindungsgemäß ist es jedoch grundsätzlich möglich, dass der mittige Netzabschnitt und die beiden seitlichen Netzabschnitte die gleiche Maschenstruktur, insbesondere die gleiche lichte Maschenweite und/oder die gleiche Maschendichte, besitzen.

Das Netz besitzt in einer weiteren Ausführungsform Bereiche mit unterschiedlichen Flächengewichten. Vorzugsweise besitzt der mittige Netzabschnitt ein kleineres Flächengewicht als die beiden seitlichen Netzabschnitte. Ein leichtgewichtiger mittiger Netzabschnitt hat den Vorteil, dass sich hierdurch Gewebeerosionen, insbesondere bei zu stützenden Organen bzw. Organteilen, reduzieren lassen. Grundsätzlich können die beiden seitlichen Netzabschnitte ein unterschiedliches Flächengewicht besitzen. Vorzugsweise besitzen die beiden seitlichen Netzabschnitte jedoch das gleiche Flächengewicht. Der mittige Netzabschnitt weist mit besonderem Vorteil ein Flächengewicht < 45 g/m², vorzugsweise < 40 g/m², insbesondere zwischen 25 und 39 g/m², bevorzugt 30 und 39 g/m², auf. Die beiden seitlichen Netzabschnitte besitzen vorzugsweise jeweils ein Flächengewicht > 40 g/m², insbesondere zwischen 41 und 90 g/m², insbesondere 42 und 75 g/m², bevorzugt 43 und 60 g_{/}m².

In einer besonders bevorzugten Ausführungsform ist das Netz, insbesondere der mittige Netzabschnitt und/oder zumindest einer der beiden seitlichen Netzabschnitte, vorzugsweise beide seitlichen Netzabschnitte, aus einem Netz gebildet bzw. hergestellt, welches unter der Bezeichnung Optilene® Mesh LP und/oder Optilene® Mesh Elastic jeweils kommerziell von der Anmelderin vertrieben wird. In einer weitergehenden Ausführungsform ist der mittige Netzabschnitt aus dem Netz Optilene^{®} Mesh LP gebildet bzw. hergestellt. Die beiden seitlichen Netzabschnitte sind jeweils vorzugsweise aus dem Netz Optilene^{®} Mesh Elastic gebildet bzw. hergestellt. Im Falle von Optilene^{®} Mesh LP handelt es sich um ein Netz aus monofilem Polypropylen mit einem Flächengewicht von 36 +/- 5 g/m², einer Maschengröße von ca. 1 mm und einer Maschendichte von ca. 112 Maschen pro cm². Das Netz Optilene^{®} Mesh Elastic ist ein Netz aus monofilem Polypropylen mit einem Flächengewicht von 48 +/- 7 g/m², einer Maschengröße von 3,6 x 2,8 mm und einer Maschendichte von ca. 60 pro cm². Grundsätzlich kann das erfindungsgemäße Netz, insbesondere der mittige Netzabschnitt und/oder zumindest einer der beiden seitlichen Netzabschnitte, vorzugsweise beide seitlichen Netzabschnitte, aus einem Netz gebildet sein, dessen Netzstruktur mit der von Optilene^{®} Mesh LP und/oder Optilene^{®} Mesh Elastic übereinstimmt, wobei das Netzmaterial ein anderes als Polypropylen, vorzugsweise resorbierbar, ist.

In einer weiteren Ausführungsform ist der mittige Netzabschnitt, vorzugsweise entlang seiner Längsränder, mit den beiden seitlichen Netzabschnitten, vorzugsweise mit jeweils einem Längsrand der beiden seitlichen Netzabschnitte, textil verbunden. Die textile Verbindung kann über einen Teil der Ränder oder über deren gesamten Länge ausgebildet sein. Vorzugsweise sind textile Verbindungen zwischen dem mittigen Netzabschnitt und den beiden seitlichen Netzabschnitten in die die textile Struktur der Netzabschnitte eingebunden. Des Weiteren können die textilen Verbindungen resorbierbar ausgebildet sein. Geeignete textile Verbindungen können auf einem Teilschussfaden und/oder auf Randfäden des mittigen Netzabschnittes und/oder zumindest eines der beiden seitlichen Netzabschnitte, vorzugsweise der beiden seitlichen Netzabschnitte, beruhen. Dies ist besonders dann von Vorteil, wenn die beiden seitlichen Netzabschnitte aus einem resorbierbaren Material gebildet sind.

Das Netz, insbesondere der mittige Netzabschnitt und/oder zumindest einer der beiden seitlichen Netzabschnitte, vorzugsweise beide seitlichen Netzabschnitte, weist in einer weiteren Ausführungsform Fäden mit einer Porosität (poröse Fäden) auf. Derartige Fäden lassen sich beispielsweise mittels Auslaugungstechniken, sogenannten Leaching-Techniken, herstellen. Beispielsweise können einer Polymerschmelze Salze hinzugegeben werden, ehe die Schmelze zu Fäden ausgesponnen oder extrudiert wird. Nach Abkühlung und Verfestigung der ausgesponnenen bzw. extrudierten Fäden können die Salze mittels eines geeigneten Lösungsmittels aus den Fäden herausgelöst werden. Die Verwendung von porösen Fäden zur Herstellung des Netzes bietet unter anderem den Vorteil einer einfacheren Wirkstoffbeladung des Netzes. Bezüglich geeigneter Wirkstoffe wird auf die folgenden Ausführungen verwiesen.

In einer weiteren Ausführungsform weist das Netz, insbesondere der mittige Netzabschnitt und/oder zumindest einer der beiden seitlichen Netzabschnitte, vorzugsweise beide seitlichen Netzabschnitte, resorbierbare Ränder auf. Dadurch können Schmerzen des Patienten, welche gegebenenfalls bei Zugbeanspruchungen des Netzes im Körper eines Patienten auftreten können, vermieden bzw. zumindest minimiert werden. Die Resorbierbarkeit der Ränder beruht dabei in der Regel auf der Verwendung von resorbierbaren Fadenmaterialien.

Zumindest einer der beiden seitlichen Netzabschnitte, vorzugsweise beide seitlichen Netzabschnitte, können versteift ausgebildet sein. Üblicherweise beruht die Versteifung auf einem Nachbehandlungsschritt des zumindest einen bzw. der beiden seitlichen Netzabschnitte. Beispielsweise kann der zumindest eine seitliche Netzabschnitt bzw. können die beiden seitlichen Netzabschnitte thermofixiert und/oder verschweißt, insbesondere ultraschallverschweißt, vorliegen. Dabei kann der zumindest eine seitliche Netzabschnitt bzw. können die beiden seitlichen Netzabschnitte mit biologischen Materialien wie zum Beispiel Gelatine, Kollagen und/oder Albumin verschweißt sein. Zum Verschweißen werden bevorzugt Lösungen der biologischen Materialien eingesetzt.

In einer weiteren Ausführungsform ist das Netz, insbesondere der mittige Netzabschnitt und/oder zumindest einer der beiden seitlichen Netzabschnitte, vorzugsweise die beiden seitlichen Netzabschnitte, als Gewirk, insbesondere Kettengewirk, ausgebildet. Vorzugsweise ist das erfindungsgemäße Netz einstückig bzw. monolithisch als Gewirk ausgebildet bzw. gefertigt. Des Weiteren kann das Netz, insbesondere der mittige Netzabschnitt und/oder zumindest einer der beiden seitlichen Netzabschnitte, vorzugsweise die beiden seitlichen Netzabschnitte, als Velours, insbesondere Veloursgewirk, ausgebildet sein. Unter einem "Velours" im Sinne der vorliegenden Erfindung soll ein Textil mit Fadenschlingen und/oder geöffneten, vorzugsweise aufgeschnittenen, Fadenschlingen, insbesondere in Form eines Flors, verstanden werden. Ein Flor bzw. Florfäden und/oder Fadenschlingen ermöglichen mit besonderem Vorteil eine stärkere Fixierung bzw. Aufhängung des erfindungsgemäßen Netzes. Ein Flor bzw. Florfäden im Sinne der vorliegenden Erfindung stehen bevorzugt rechtwinklig oder in einem spitzen Winkel von der Netzoberfläche ab. Grundsätzlich kann ein Flor bzw. können Florfäden aber auch im Wesentlichen horizontal zur Netzoberfläche verlaufend ausgebildet sein. Bei dem Velours, insbesondere Veloursgewirk, kann es sich um Einfach- oder Doppelvelours, insbesondere Einfachvelours- oder Doppelveloursgewirks, handeln. Im Falle eines Doppelvelours, insbesondere Doppelveloursgewirk, können unterschiedliche Flor- bzw. Polhöhen, insbesondere auf gegenüberliegenden Flächenseiten des Netzes, insbesondere des mittigen Netzabschnittes und/oder zumindest eines der beiden seitlichen Netzabschnitte, vorzugsweise der beiden seitlichen Netzabschnitte, ausgebildet sein.

Ist das Netz, insbesondere der mittige Netzabschnitt und/oder zumindest einer der beiden seitlichen Netzabschnitte, vorzugsweise die beiden seitlichen Netzabschnitte, veloursartig ausgebildet, sind die beiden seitlichen Netzabschnitte vorzugsweise resorbierbar oder teilresorbierbar ausgebildet. Sind die beiden seitlichen Netzabschnitte teilresorbierbar ausgebildet, ist es bevorzugt, wenn lediglich Veloursschlingen und/oder durch Öffnen, insbesondere Aufschneiden, von Veloursschlingen gebildete Veloursfäden der beiden seitlichen Netzabschnitte resorbierbar ausgebildet sind und die beiden seitlichen Netzabschnitte im Übrigen nicht resorbierbar ausgebildet sind. Die Veloursschlingen und/oder Veloursfäden erleichtern zum Einen mit besonderem Vorteil die Verankerung des Netzes. Zum anderen wird durch die Resorbierbarkeit der Veloursschlingen und/oder Veloursfäden der Anteil an im Körper eines Patienten zurückbleibendem Fremdmaterial verringert, wodurch das Wohlbefinden des Patienten deutlich verbessert werden kann.

In einer weitergehenden Ausführungsform besitzt der mittige Netzabschnitt eine gewirkte und vorzugsweise veloursfreie Netzstruktur. Bei der Netzstruktur kann es sich beispielsweise um die bereits beschriebene Struktur von Optilene^{®} Mesh LP handeln. Zumindest einer der beiden seitlichen Netzabschnitte, bevorzugt beide seitlichen Netzabschnitte, besitzt vorzugsweise eine Veloursstruktur, insbesondere gewirkte Veloursstruktur, mit Fadenschlingen und/oder geöffneten, vorzugsweise aufgeschnittenen, Fadenschlingen (Flor). Wie bereits erwähnt, kann es bevorzugt sein, wenn die beiden seitlichen Netzabschnitte teilresorbierbar ausgebildet sind, wobei bevorzugt lediglich die Fadenschlingen und/oder geöffneten, vorzugsweise aufgeschnittenen, Fadenschlingen (Flor bzw. Florfäden) resorbierbar ausgebildet sind. Der zumindest eine seitliche Netzabschnitt bzw. die beiden seitlichen Netzabschnitte können einen variablen, insbesondere graduell ausgebildeten, Veloursanteil und/oder eine variable, insbesondere graduell ausgebildete, Veloursgröße besitzen.

Erfindungsgemäß kann es weiterhin vorgesehen sein, dass Fadenschlingen, insbesondere Veloursschlingen, der beiden seitlichen Netzabschnitte umgeschlagen und fixiert sind. Auf diese Weise können die seitlichen Netzabschnitte verstärkt bzw. stabilisiert werden. Dies gilt auch für aus den seitlichen Netzabschnitten gebildete Fortsätze des im Folgenden noch näher beschriebenen chirurgischen Implantats.

In einer weiteren Ausführungsform sind zumindest die beiden seitlichen Netzabschnitte, insbesondere die beiden seitlichen Netzabschnitte, als dreidimensionales Textil, insbesondere Abstandstextil, ausgebildet. Entsprechende Netzabschnitte bzw. hieraus gebildete Fortsätze eines im Folgenden noch näher beschriebenen chirurgischen Implantats lassen sich aufgrund der dickeren Ausgestaltung als Abstandstextil besser fixieren, wodurch die Fixierung bzw. Aufhängung des Netzes bzw. eines hieraus hergestellten Implantats ebenso verbessert wird. Bevorzugt sind die beiden seitlichen Netzabschnitte als dreidimensionales Gewirk ausgebildet. Bei dem mittigen Netzabschnitt handelt es sich vorzugsweise um ein veloursfreies und insbesondere gewirktes Netz, beispielsweise um das bereits erwähnte Optilene^{®} Mesh LP. Zur besseren Aufhängung des Netzes bzw. eines hieraus hervorgehenden Implantats kann die dreidimensionale Textilstruktur der beiden seitlichen Netzabschnitte umgeschlagen und fixiert vorliegen. Die umgeschlagene und fixierte dreidimensionale Textilstruktur kann als Ankerstruktur zur Verankerung des Netzes bzw. eines hieraus hergestellten Implantats im Körper eines Patienten dienen. Die Ausbildung einer vorzugsweise dreidimensionalen Ankerstruktur hat den Vorteil, dass durch Umschlagen der dreidimensionalen Struktur die Fixierungslänge für den mittigen Netzabschnitt deutlich verkürzt werden kann.

Erfindungsgemäß kann es weiterhin vorgesehen sein, dass das dreidimensionale Textil, insbesondere Verbindungsfäden eines Abstandstextils, teilweise aufgeschnitten und insbesondere einseitig oder zweiseitig umgeklappt sind. Dies führt zu einer stärkeren Fixierung des Netzes bzw. eines aus dem Netz hergestellten chirurgischen Implantats durch abstehende Fäden, insbesondere Monofilamente.

Die in den vorhergehenden Ausführungsformen erwähnten Fadenschlingen, insbesondere Velourschlingen, und/oder geöffneten, vorzugsweise aufgeschnittenen, Fadenschlingen, insbesondere Veloursschlingen (Florfäden), können zusätzlich versteift, beispielsweise thermofixiert und/oder verschweißt, insbesondere ultraschallverschweißt, vorliegen. Insbesondere können die Fadenschlingen mit biologischen Materialien verschweißt sein. Die Fadenschlingen und/oder die geöffneten Fadenschlingen können weiterhin resorbierbar oder nicht resorbierbar ausgebildet sein. Bevorzugt ist es, wenn die Fadenschlingen und/oder die geöffneten Fadenschlingen resorbierbar sind.

Das Netz, insbesondere der mittige Netzabschnitt und/oder zumindest einer der beiden seitlichen Netzabschnitte, vorzugsweise die beiden seitlichen Netzabschnitte, ist in einer weiteren Ausführungsform in einer Trikot-, Trikot-Atlas-, Samt-, Tuch-, Frottierbindung oder Kombinationen davon ausgeführt.

Besonders bevorzugt ist das Netz, d.h. der mittige Netzabschnitt und die beiden seitlichen Netzabschnitte, einstückig bzw. monolithisch gewirkt ausgebildet. Mit anderen Worten ist das Netz vorzugsweise in einem Arbeitsgang an einer Wirkmaschine hergestellt, vorzugsweise in der Abfolge seitlicher Netzabschnitt - mittiger Netzabschnitt - seitlicher Netzabschnitt. Eine Verbindung der Netzabschnitte untereinander kann, wie bereits erwähnt, beispielsweise auf Teilschussfäden und/oder Randfäden der Netzabschnitte basieren.

In einer möglichen Ausführungsform ist das Netz aus drei eigenständigen Einzelnetzen, wovon ein Einzelnetz als mittiger Netzabschnitt und die beiden übrigen Einzelnetze als seitliche Netzabschnitte beidseits des mittigen Netzabschnittes verwendet werden, zusammengesetzt. In diesem Fall kann eine Verbindung der Einzelnetze bzw. der Netzabschnitte beispielsweise auf einer Verklebung, Verschweißung, insbesondere Ultraverschweißung, Thermofixierung oder auf rein mechanischen Verbindungen beruhen. Eine Verklebung der Einzelnetze kann beispielsweise mittels Klebstoffzusammensetzungen auf Basis von Cyanoacrylat-Monomeren durchgeführt werden.

Das Netz, insbesondere der mittige Netzabschnitt und/oder zumindest einer der beiden seitlichen Netzabschnitte, vorzugsweise die beiden seitlichen Netzabschnitte, kann monofile Fäden, bevorzugt monofile Polyolefinfäden, insbesondere monofile Polypropylenfäden, und/oder multifile Fäden aufweisen.

Das Netz, insbesondere der mittige Netzabschnitt und/oder zumindest einer der beiden seitlichen Netzabschnitte, vorzugsweise die beiden seitlichen Netzabschnitte, weist in einer weitergehenden Ausführungsform Monofilamentgarne, insbesondere monofile Polyolefingarne, vorzugsweise monofile Polypropylengarne auf.

Bevorzugt ist das Netz, insbesondere der mittige Netzabschnitt und/oder zumindest einer der beiden seitlichen Netzabschnitte, vorzugsweise die beiden seitlichen Netzabschnitte, aus monofilen Fäden, insbesondere monofilen Polyolefinfäden, vorzugsweise monofilen Polypropylenfäden, gebildet.

Des Weiteren kann es insbesondere bevorzugt sein, wenn das Netz, insbesondere der mittige Netzabschnitt und/oder zumindest einer der beiden seitlichen Netzabschnitte, vorzugsweise die beiden seitlichen Netzabschnitte, aus Monofilamentgarnen, insbesondere monofilen Polyolefingarnen, bevorzugt monofilen Polypropylengarnen, gebildet ist. Bevorzugt weist das Netz, besonders bevorzugt der mittige Netzabschnitt, antimikrobiell, insbesondere antibakteriell, ausgerüstete Fäden auf. Bezüglich geeigneter antimikrobieller bzw. antibakterieller Wirkstoffe wird auf die im Folgenden noch aufgeführten Wirkstoffe verwiesen.

In einer weiteren Ausführungsform weist das Netz, insbesondere der mittige Netzabschnitt und/oder zumindest einer der beiden seitlichen Netzabschnitte, vorzugsweise die beiden seitlichen Netzabschnitte, auf zumindest einer Flächenseite davon, insbesondere nur auf einer Flächenseite davon, eine Beschichtung aus einem bioverträglichen Material auf. Das Material ist vorzugsweise resorbierbar. Geeignete Beschichtungsmaterialien können aus der Gruppe bestehend aus Gelatine, Collagen, Retikulin, Elastin, Fibronektin, Albumin, Methylcellulose, Hydroxymethylcellulose, Hydroxyethylcellulose, Hydroxypropylcellulose, Hydroxybutylcellulose, Hydroxyethylmethylcellulose, Hydroxypropylmethylcellulose, Carboxymethylcellulose, Chitosan, Dermatansulfat, Keratansulfat, Chondroitinsulfat, Hyaluronsäure, Stärke, Amylopektin, Amylose, Dextran, Polyvinylalkohol, Salze davon und Kombinationen davon ausgewählt sein. Des Weiteren kann die Beschichtung ein biologisches, insbesondere tierisches, Gewebe aufweisen. Das Gewebe kann xenogenen, insbesondere porcinen, bovinen oder equinen, Ursprungs sein. Bevorzugt ist das Gewebe ein Pericardgewebe. Die Beschichtung ist vorzugsweise glatt und insbesondere abdichtend ausgebildet. Die Beschichtung kann insbesondere als Folie oder Film ausgebildet sein. Mittels einer Beschichtung, wie sie in diesem Absatz beschrieben ist, können in vorteilhafter Weise unerwünschte Gewebeadhäsionen oder -erosionen vermieden werden.

In einer weiteren Ausführungsform weist das Netz, insbesondere der mittige Netzabschnitt und/oder zumindest einer der beiden seitlichen Netzabschnitte, vorzugsweise die beiden seitlichen Netzabschnitte, eine dreidimensionale Textilstruktur, vorzugsweise für Körperzellen und/oder Körpergewebe, insbesondere Bindegewebe, hintergreifbare Strukturen, auf. Die hintergreifbaren Strukturen können resorbierbar ausgebildet sein. Dadurch können mittel- bis langfristige Gewebereizungen aufgrund von möglichen Relativbewegungen zwischen dem Netz und den zu stützenden prolapierten Organen bzw. Organteilen vermieden werden. Bei den hintergreifbaren Strukturen kann es sich beispielsweise um Florfäden, Fransen, texturierte Fäden, Widerhaken, pilzkopfartige Fadenstrukturen, Kantenfäden, Randfäden, Veloursschlingen, Flottungen oder Kombinationen davon handeln. Die hintergreifbaren Strukturen können durch Laserschneiden erzeugt und/oder zusätzlich versteift vorliegen, beispielsweise durch eine Thermobehandlung oder eine Ultraschallbehandlung.

Erfindungsgemäß kann es weiterhin vorgesehen sein, dass eine Flächenseite, in der Regel eine Hauptflächenseite, des Netzes, insbesondere des mittigen Netzabschnittes und/oder zumindest eines der beiden seitlichen Netzabschnitte, vorzugsweise der beiden seitlichen Netzabschnitte, eine dreidimensionale Textilstruktur bzw. für Körperzellen und/oder Körpergewebe hintergreifbare Strukturen aufweist und die gegenüberliegende Flächenseite eine Beschichtung aufweist, wie sie beispielsweise im vorherigen Absatz beschrieben ist.

Das Netz, insbesondere der mittige Netzabschnitt und/oder zumindest einer der beiden seitlichen Netzabschnitte, vorzugsweise die beiden seitlichen Netzabschnitte, kann zusätzlich Additive, insbesondere biologische, medizinische oder pharmazeutische Wirkstoffe, aufweisen. Vorteilhafte Additive können zum Beispiel aus der Gruppe bestehend aus antimikrobielle, insbesondere antibakterielle, Wirkstoffe, desinfizierende Wirkstoffe, entzündungshemmende Wirkstoffe, schmerzlindernde Wirkstoffe, geruchsbekämpfende Wirkstoffe, zelluläre Wachstumsfaktoren, zelluläre Rekrutierungsfaktoren, zelluläre Differenzierungsfaktoren, zelluläre Adhäsionsfaktoren und Kombinationen davon ausgewählt sein. Unter antimikrobiellen Wirkstoffen sollen im Sinne der vorliegenden Erfindung Wirkstoffe verstanden werden, welche die Vermehrungsfähigkeit und/oder die Infektiosität von Mikroorganismen, insbesondere Bakterien, hemmen bzw. verringern und/oder Mikroorganismen, insbesondere Bakterien, abtöten.

Erfindungsgemäß ist es besonders bevorzugt, wenn das Netz, insbesondere der mittige Netzabschnitt und/oder zumindest einer der beiden seitlichen Netzabschnitte, vorzugsweise die beiden seitlichen Netzabschnitte, mit einem antimikrobiellen, insbesondere antibakteriellen, Wirkstoff oder einer antimikrobiellen, insbesondere antibakteriellen, Wirkstoffkombination, ausgerüstet ist. Bevorzugte antimikrobielle bzw. antibakterielle Wirkstoffe sind Metalle, Metalllegierungen und/oder Metallverbindungen, insbesondere Metallsalze. Beispielsweise können geeignete Wirkstoffe aus der Gruppe bestehend aus Kupfer, Zink, Silber, Gold, Platin, Titan, Kombinationen davon und Verbindungen, insbesondere Salze, davon ausgewählt sein. Des Weiteren kann es sich bei den antimikrobiellen bzw. antibakteriellen Wirkstoffen um organische Verbindungen, vorzugsweise aus der Gruppe bestehend aus Chitosan, Poly-s-Lysin, Polyhexamethylenbiguanid, Akazid^{®}, Cyclohexidin, Cetyltrimethylammoniumbromid (CTAB), Octinidin, Benzalkoniumchlorid, Cetylpyridiniumchlorid, Salze, insbesondere Fettsäuresalze, davon und Kombinationen davon, handeln.

Die Wirkstoffe können in einer weiteren Ausführungsform als Partikel, insbesondere Mikropartikel und/oder Nanopartikel, oder in Form einer Beschichtung, beispielsweise durch Eintauchen des Netzes in eine entsprechende Wirkstofflösung und/oder durch Besprühen des Netzes mit einer entsprechenden Wirkstofflösung, vorliegen.

Bevorzugt liegt das Netz in sterilisierter und insbesondere konfektionierter Form vor.

Das textile Netz der vorliegenden Erfindung eignet sich in besonderer Weise als chirurgisches Implantat zur Beckenbodenrekonstruktion, vorzugsweise zur Versorgung oder Behandlung von Prolapsen bzw. prolapsartigen Erkrankungen. Besonders bevorzugt ist das textile Netz als Implantat zur Versorgung bzw. Behandlung von Prolapsen, Zystozelen, Rektozelen, Douglasozelen oder Enterozelen ausgebildet. Bei den zu versorgenden bzw. behandelnden Prolapsen kann es sich insbesondere um Anal-, Rektum-, Scheide- oder Uterusprolapse handeln. Des Weiteren eignet sich das Netz auch als Implantat zur Vorbeugung von Dyspareunia. Grundsätzlich kann das Netz aber auch als Herniennetz oder zur Behandlung der Harninkontinenz verwendet werden.

Das Netz sowie das im Folgenden noch näher beschriebene Implantat können mittels Klebe- und/oder Nähtechniken im Körper eines Patienten fixiert werden. Insbesondere kann es vorgesehen sein, dass das Netz bzw. Implantat durch Annähen im Körper des Patienten fixiert und der Mittelabschnitt des Netzes bzw. der Grundkörper des im Folgenden noch eingehender beschriebenen Implantats zusätzlich durch ein Verkleben fixiert wird. Bevorzugte Klebetechniken beruhen auf dem Einsatz von Gewebeklebern, beispielsweise Fibrinklebern und/oder Klebern auf Basis von Cyanoacrylat-Monomeren.

Weiterhin kann das Netz indikationsspezifisch modifiziert, insbesondere zurechtgeschnitten oder zurechtgestanzt, werden.

Ein weiterer Aspekt der vorliegenden Erfindung betrifft ein chirurgisches Implantat, insbesondere zur Beckenbodenrekonstruktion, vorzugsweise zur Versorgung oder Behandlung von Prolapsen oder prolapsartigen Erkrankungen, oder zur Vermeidung von Dyspareunia, umfassend einen Grundkörper und Fortsätze, vorzugsweise streifenförmige Fortsätze. Das Implantat ist vorzugsweise zumindest teilweise, insbesondere nur teilweise, anisotrop elastisch ausgebildet. Der Grundkörper und/oder die Fortsätze besitzen bevorzugt eine Netzstruktur, insbesondere eine textile Netzstruktur. Der Grundkörper und/oder die Fortsätze, bevorzugt Netzstrukturen davon, können resorbierbar, teilresorbierbar und/oder nicht resorbierbar ausgebildet sein. Bevorzugt ist der Grundkörper nicht resorbierbar ausgebildet. Die Fortsätze sind vorzugsweise resorbierbar ausgebildet.

Um unnötige Wiederholungen zu vermeiden, wird bezüglich weiterer Merkmale und Vorteile zu der Netzstruktur des Grundkörpers sowie der Fortsätze vollständig und ausdrücklich auf die vorangegangenen Ausführungen zu dem mittigen Netzabschnitt sowie den beiden seitlichen Netzabschnitten des erfindungsgemäßen Netzes Bezug genommen.

Bezüglich weiterer Merkmale und Vorteile des Implantats wird auf die noch im Folgenden gemachten Ausführungen verwiesen.

Des Weiteren wird auch ein Verfahren zur Herstellung eines chirurgischen Implantats, insbesondere zur Beckenbodenrekonstruktion, vorzugsweise zur Versorgung oder Behandlung von Prolapsen oder prolapsartigen Erkrankungen, oder zur Vermeidung von Dyspareunia, erläutert. Hierzu wird das Implantat aus einem Netz gemäß der vorliegenden Erfindung, insbesondere einer Netzbahn davon, hergestellt bzw. angefertigt.

Bezüglich weiterer Merkmale und Vorteile zu dem Verfahren wird auf die noch im Folgenden gemachten Ausführungen vollständig Bezug genommen.

Ein weiterer Aspekt der vorliegenden Erfindung betrifft ein chirurgisches Implantat, insbesondere zur Beckenbodenrekonstruktion, vorzugsweise zur Versorgung oder Behandlung von Prolapsen oder prolapsartigen Erkrankungen, oder zur Vermeidung von Dyspareunia, welches aus einem Netz gemäß der vorliegenden Erfindung, insbesondere einer Netzbahn davon, hergestellt bzw. angefertigt ist. In der Regel wird das chirurgische Implantat aus dem textilen Netz gemäß der vorliegenden Erfindung, insbesondere einer Netzbahn davon, herausgeschnitten oder -gestanzt. Das Herausschneiden kann beispielsweise mittels eines Lasers vorgenommen werden. Es kann daher vorgesehen sein, dass das chirurgische Implantat laserbeschnittene Kanten aufweist.

Das Implantat basiert vorzugsweise auf einem Grundkörper und Fortsätzen. Die Fortsätze weisen in der Regel von dem Grundkörper weg. Bevorzugt stehen die Fortsätze seitlich von dem Grundkörper ab.

Das Implantat, vorzugsweise dessen Fortsätze, können Befestigungsmittel zum Befestigen an ein chirurgisches Einführinstrument, vorzugsweise an eine chirurgische Nadel, aufweisen. Bei den Befestigungsmitteln kann es sich beispielsweise um eine Öffnung handeln, welche eine Befestigung mit dem chirurgischen Einführinstrument auf Basis eines einfachen Auf- oder ein Einhängmechanismus erlaubt. Alternativ oder in Kombination können die Befestigungsmittel auch textil ausgebildet sein.

In einer weitergehenden Ausführungsform sind der Grundkörper und die Fortsätze integral bzw. monolithisch miteinander verbunden. Dies hat den Vorteil, dass nachträgliche Bearbeitungsschritte zur Verbindung von Grundkörper und Fortsätze entfallen.

Es kann grundsätzlich aber auch vorgesehen sein, den Grundkörper und die Fortsätze erst im Rahmen eines nachträglichen Bearbeitungsschrittes mittels geeigneter Fügetechniken, beispielsweise Verkleben, Verschweißen, insbesondere Ultraschallverschweißen, und/oder Vernähen, zu einem einstückigen Implantat zu verbinden. Geeignete Klebetechniken können beispielsweise die Verwendung von Klebstoffzusammensetzungen auf Basis von Cyanoacrylat-Monomeren vorsehen. Weitere geeignete Verbindungstechniken können auf Veloursverschlüssen, Klettverschlüssen, Knopflochverschlüssen, Druckknopfverschlüssen, Schlitzverschlüssen oder auf Verschlüssen, welche nach dem sogenannten Mädchenfänger-Prinzip funktionieren, beruhen. Verbindungsstellen bzw. Verbindungselemente, welche eine Verbindung zwischen Grundkörper und Fortsätzen ermöglichen, können resorbierbar, teilresorbierbar oder nicht resorbierbar sein. Die nachträgliche Verbindung von Grundkörper und Fortsätzen kann beispielsweise dann von Vorteil sein, wenn unterschiedliche Netzmaterialien für den Grundkörper und die F ortsätze zum Einsatz kommen sollen oder der Grundkörper und/oder die Fortsätze in Form und/oder Größe von einem Chirurgen angepasst und zurechtgeschnitten werden. Mit anderen Worten kann es erfindungsgemäß vorgesehen sein, dass der Grundkörper und die Fortsätze erst von einem Chirurgen zu einem einsatzfähigen Implantat verbunden werden.

In einer besonders bevorzugten Ausführungsform ist das Implantat, insbesondere der Grundkörper und/oder die Fortsätze, aus einem erfindungsgemäßen Netz bzw. einer Netzbahn davon hergestellt bzw. angefertigt. Der Grundkörper ist vorzugsweise aus dem mittigen Netzabschnitt und die Fortsätze sind vorzugsweise aus den beiden seitlichen Netzabschnitten des erfindungsgemäßen Netzes gebildet. Besonders bevorzugt ist der Grundkörper dabei elastischer ausgebildet als die Fortsätze bzw. umgekehrt sind die Fortsätze weniger elastisch ausgebildet als der Grundkörper.

Der Grundkörper kann eine kreisförmige, ellipsoide, ovale oder polygone Form besitzen. Zum Beispiel kann der Grundkörper eine dreieckige, viereckige, fünfeckige bzw. pentagonale und/oder sechseckige bzw. hexagonale Form besitzen. Beispiele für viereckige Formen sind Quadrate, Rechtecke, Rauten, Parallelogramme oder Trapeze. Erfindungsgemäß kann es bevorzugt sein, wenn der Grundkörper trapezförmig ausgebildet ist. Die Fortsätze können an den Ecken eines polygonen Grundkörpers ausgebildet sein. In der Regel sind die Fortsätze streifenförmig ausgebildet. Die Fortsätze können abgerundete oder sich V-förmig verjüngende Enden aufweisen. Des Weiteren können die Fortsätze an ihren Enden Aussparungen, insbesondere Löcher, zur Befestigung mit einem chirurgischen Einführinstrument, insbesondere einer chirurgischen Nadel, aufweisen.

Das Implantat kann grundsätzlich eine Vielzahl von Fortsätzen besitzen. Typischerweise besitzt das Implantat eine gerade Anzahl von Fortsätzen, beispielsweise zwei, vier, sechs oder acht Fortsätze.

In einer weiteren Ausführungsform weist das Implantat Fortsätze mit unterschiedlichen Formen und/oder Größen auf.

Zur Verbesserung der Fixierung bzw. Aufhängung des Implantats im Körper eines Patienten können die Fortsätze an ihren freien Enden aufgeschnitten und ein- oder zweiseitig, insbesondere unter Ausbildung von schleifen- bzw. loopförmigen Verankerungsstrukturen, umgeschlagen und fixiert vorliegen.

In einer weiteren Ausführungsform liegen Fortsätze des chirurgischen Implantats versteift vor. Geeignete Versteifungen lassen sich beispielsweise mittels thermischer Nachbehandlungen, insbesondere mittels Erhitzen und anschließendem Schrumpfen, und/oder Verschweißungen, insbesondere Ultraschallverschweißungen, der Fortsätze erzielen.

Wie bereits erwähnt, sind die Fortsätze vorzugsweise weniger elastisch ausgebildet als der Grundkörper des Implantats. Überraschenderweise hat sich insoweit herausgestellt, dass die Elastizität der Fortsätze abhängig vom Winkel ist, um den die Fortsätze gegenüber der Querrichtung des Grundkörpers geneigt sind bzw. abstehen. Dieser Winkel wird im Folgenden als Winkel α bezeichnet. In der Regel stimmt die Querrichtung des Grundkörpers mit der Längsrichtung einer Netzbahn überein, aus der ein erfindungsgemäßes Implantat herausgeschnitten werden kann. Bei dem Winkel α handelt es sich in der Regel um einen spitzen Winkel. Bevorzugt weisen die Fortsätze einen Winkel α < 70 Grad, vorzugsweise zwischen 30 und 60 Grad, auf. Implantate, deren Fortsätze in einem Winkel ≤ 60 Grad gegenüber der Querrichtung des Grundkörpers abstehen, sind besonders bevorzugt, da in diesem Fall die Elastizität der Fortsätze in Längsrichtung signifikant geringer ist als die Elastizität des Grundkörpers in Längsrichtung. In besonders bevorzugten Ausführungsformen weisen die Fortsätze des Implantats einen Winkel α zwischen 30 und 45 Grad, insbesondere 30 und 35 Grad, auf. Besonders bevorzugt ist dabei ein Winkel α der Fortsätze von ca. 30 Grad, da in diesem Fall die Fortsätze in deren Längsrichtung sogar eine geringere bzw. niedrigere Elastizität aufweisen als der Grundkörper in dessen Querrichtung.

Die bezüglich des erfindungsgemäßen Netzes beschriebenen Ausführungsformen und Vorteile gelten sinngemäß auch für das chirurgische Implantat.

So kann beispielsweise der Grundkörper eine gewirkte und vorzugsweise veloursfreie Netzstruktur besitzen. Die Fortsätze können jeweils eine Veloursstruktur, insbesondere eine Doppelveloursstruktur, besitzen. Die Veloursstrukturen können Fadenschlingen und/oder aufgeschnittene Fadenschlingen, d.h. einen sogenannten Flor bzw. Florfäden, aufweisen, welche mit besonderem Vorteil eine verbesserte Fixierung des Implantats über dessen Fortsätze erlauben. Zur Verstärkung bzw. Stabilisierung der Fortsätze können abstehende Fäden bzw. Fadenenden, insbesondere Florfäden, der Fortsätze umgeschlagen und fixiert sein. Weiterhin können die Fortsätze eine dreidimensionale Textilstruktur besitzen. Durch die dreidimensionale Textilstruktur sind die Fortsätze vorzugsweise dicker als der Grundkörper ausgebildet, was ebenfalls zu einer verbesserten Fixierung des chirurgischen Implantats führt. Beispielsweise können die Fortsätze als dreidimensionales Gewirk ausgebildet sein. Besonders vorteilhaft ist es, wenn die Fortsätze eine Abstandstextilstruktur, vorzugsweise eine gewirkte Abstandstextilstruktur (Abstandsgewirk), besitzen. In diesem Fall kann es zudem von Vorteil sein, wenn Verbindungsfäden der Abstandstextilstruktur aufgeschnitten und die Abstandstextilstruktur, ausgehend von den freien Enden der Fortsätze wenigstens teilweise einseitig oder zweiseitig umgeschlagen bzw. umgeklappt und fixiert sind, gegebenenfalls unter Ausbildung schleifen- bzw. loopförmiger Strukturen, welche mit Vorteil zur Aufhängung oder Fixierung des Implantats im Körper eines Patienten dienen können. Die in diesem Fall von den Fortsätzen abstehenden Fäden bzw. Fadenenden, insbesondere Monofilamente bzw. Monofilamentenden, bewirken ebenfalls eine stärkere Fixierung bzw. Aufhängung des Implantats im Körper eines Patienten.

Des Weiteren kann es sich bei der im vorherigen Absatz erwähnten dreidimensionalen Textilstruktur der Fortsätze auch für Körperzellen und/oder Körpergewebe hintergreifbare Strukturen handeln. Insoweit wird vollständig auf die bisherige Beschreibung verwiesen.

Bezüglich weiterer Merkmale und Vorteile des Implantats wird vollständig auf die vorangegangenen Ausführungen zu dem erfindungsgemäßen Netz Bezug genommen.

Ein weiterer Aspekt betrifft ein chirurgisches Kit zur Versorgung oder Behandlung von Prolapsen, prolapsartigen Erkrankungen oder zur Vermeidung von Dyspareunia, umfassend zumindest ein chirurgisches Implantat gemäß der vorliegenden Erfindung. Bevorzugt umfasst das Kit zwei, drei oder mehr chirurgische Implantate. Die Implantate unterscheiden sich vorzugsweise hinsichtlich ihrer Größe und/oder Form. Insbesondere kann das Kit unterschiedliche Größen und/oder Formen für einen Grundkörper und/oder für Fortsätze enthalten, welche abhängig von der im Einzelfall zu versorgenden bzw. behandelnden Körperdefektzone zu einem chirurgischen Implantat gewünschter Größe und/oder Form verbunden werden können. Zur Verbindung von Grundkörpern und Fortsätzen kann das chirurgische Kit außerdem geeignete Verbindungsmittel, beispielsweise Nahtmaterial, Kleber, insbesondere Gewebekleber, Klettverschlusselemente, Knopflochverschlusselemente, Druckknopfverschlusselemente oder dergleichen, enthalten. Des Weiteren kann das Kit zumindest ein chirurgisches Einführinstrument, in der Regel eine chirurgische Nadel, enthalten. Weiterhin kann das Kit Fixierhilfen zum Fixieren des chirurgischen Einführinstrumentes an das Implantat, insbesondere an zumindest einen Fortsatz davon, enthalten. Außerdem kann das Kit auch Fixierhilfen zur Fixierung des Implantats im Körper eines Patienten enthalten. Derartige Fixierhilfen können beispielsweise Kleber, insbesondere Gewebekleber, Nahtmaterialien, Anker und/oder Klemmen, umfassen. Der Vorteil eines chirurgischen Kits, wie er im vorliegenden Absatz beschrieben ist, liegt darin, dass er eine einfache und bequeme Anpassung des chirurgischen Implantats an patientenspezifische Körperdefektzonen, insbesondere Prolapszonen, erlaubt. Bezüglich weiterer Merkmale und Vorteile hierzu wird vollständig auf die bisherige Beschreibung Bezug genommen.

Schließlich ist auch die Verwendung des textilen Netzes zur Herstellung eines chirurgischen Implantats, insbesondere zur Beckenbodenrekonstruktion, vorzugsweise zur Versorgung oder Behandlung von Prolapsen oder prolapsartigen Erkrankungen, oder zur Vermeidung von Dyspareunia. beispielhaft beschrieben. Bezüglich weiterer Merkmale und Vorteile hierzu wird ebenso vollständig auf die bisherige Beschreibung verwiesen.

Weitere Einzelheiten und Merkmale sowie Vorteile der Erfindung ergeben sich aus der nachfolgenden Beschreibung anhand der Abbildungen in Kombination mit den Unteransprüchen. Hierbei können einzelne Merkmale der Erfindung allein oder in Kombination mit anderen Merkmalen verwirklicht sein. Die Abbildungsbeschreibungen sind dabei lediglich als eine beschreibende, keineswegs als in irgendeiner Weise limitierende Offenbarung zu verstehen. Die Abbildungen werden hiermit durch ausdrückliche Bezugnahme zum Inhalt dieser Beschreibung gemacht.

In den Abbildungen zeigen:
- Figur 1:: eine photographische Darstellung einer Ausführungsform des erfindungsgemäßen Netzes,
- Figur 2:: eine mikroskopische Aufnahme eines mittigen Netzabschnittes einer Ausführungsform des erfindungsgemäßen Netzes,
- Figur 3:: eine mikroskopische Aufnahme eines seitlichen Netzabschnittes einer Ausführungsform des erfindungsgemäßen Netzes,
- Figur 4:: ein textiles Legungsbild einer Ausführungsform des erfindungsgemäßen Netzes,
- Figur 5:: eine schematische Darstellung einer Ausführungsform des erfindungsgemäßen Netzes einschließlich den Konturen eines erfindungsgemäßen chirurgischen Implantats,
- Figur 6:: Dehnbarkeitswerte für Grundkörper und Fortsätze eines erfindungsgemäßen chirurgischen Implantats.

### Figurenbeschreibung

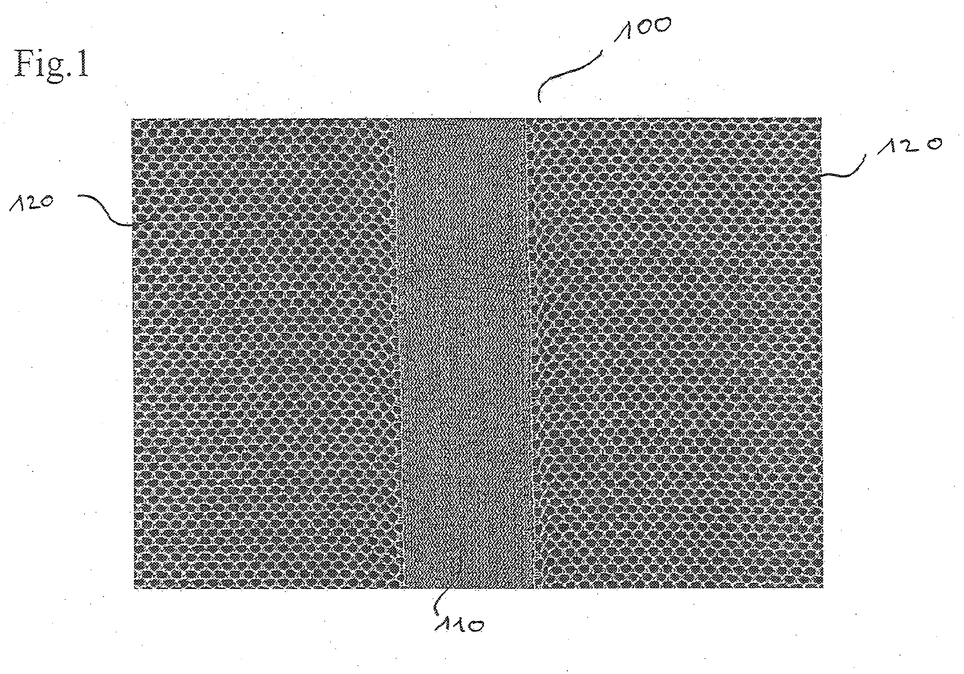

Das in der Figur 1 dargestellte Netz 100 weist einen mittigen Netzabschnitt 110 sowie zwei seitliche Netzabschnitte 120 beidseits des mittigen Netzabschnittes 110 auf. Der mittige Netzabschnitt 110 ist vorzugsweise nicht resorbierbar ausgebildet kann beispielsweise aus einem Netz mit der Bezeichnung Optilene^{®} Mesh LP gebildet sein. Die beiden seitlichen Netzabschnitte 120 können jeweils aus einem Netz mit der Bezeichnung Optilene^{®} Mesh Elastic gebildet sein. Beide Netzarten sind in der vorliegenden Beschreibung eingehend beschrieben. Besonders bevorzugt ist es, wenn die beiden seitlichen Netzabschnitte 120 aus einem resorbierbaren Material wie beispielsweise Polyhydroxyalkanoaten bzw. Copolymeren davon gebildet sind. Das Netz 100 stellt vorzugsweise eine textile Verbundstruktur aus zwei verschiedenen Netzarten dar. Derartige Verbund- bzw. Gradientennetze besitzen den Vorteil, dass textile Eigenschaften von verschiedenen Netzarten zu einem multifunktionalen Netz kombiniert sind. Das in Abbildung 1 dargestellte Netz 100 besitzt bevorzugt ein durchschnittliches Flächengewicht (über die Gesamtbreite ermittelt) von ca. 43,5 g/cm² und kann aus biologischer bzw. medizinischer Sicht als risikoarm eingestuft werden.

Allgemein können die charakteristischen textilen Eigenschaften eines Verbund- bzw. Gradientennetzes, insbesondere ein anisotropes Dehnungs- und/oder Reißkraftverhalten, gezielt durch Verwendung von Fäden mit einem unterschiedlichen Material, Titer und/oder Durchmesser und/oder durch die Erzeugung unterschiedlicher Maschenstrukturen, insbesondere hinsichtlich der lichten Maschenweite und/oder der Maschendichte, eingestellt werden. Zur Erzeugung unterschiedlicher Maschendichten können beispielsweise hierfür relevante Parameter, wie beispielsweise der Einzug und die Anzahl eingesetzter Legeschienen, an einer Textilmaschine, vorzugsweise Wirkmaschine, bewusst ausgewählt und kombiniert werden.

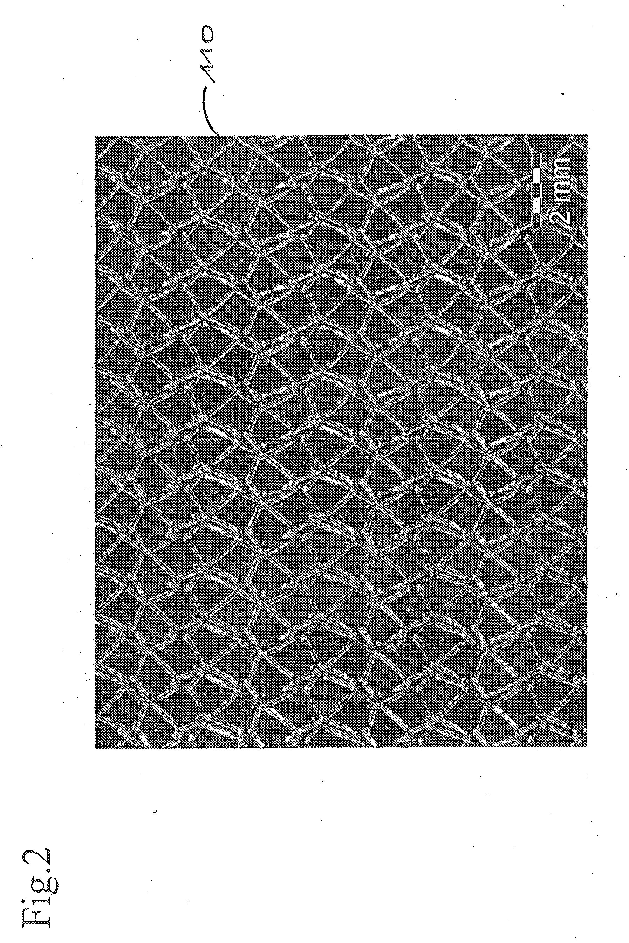

Die Figur 2 zeigt eine mikroskopische Aufnahme eines mittigen Netzabschnittes 110, der aus dem Netz Optilene^{®} Mesh LP gebildet ist. Die Netzstruktur weist eine besonders leichte und insbesondere flexible, jedoch feste Konstruktion auf. Die Struktur selbst ist aus gleichmäßigen, ineinanderhängenden Maschen mit einer lichten Maschenweite von ca. 1 mm gebildet. Des Weiteren wird die Netzstruktur aus monofilen Polypropylengarnen gebildet. Das Flächengewicht beträgt ca. 36 ± 5 g/m². Die Maschendichte liegt in Übereinstimmung mit der dichten Maschenstruktur bei ca. 112 Maschen pro cm². Die im Rahmen von Höchstzugkraftmessungen ermittelten Festigkeiten (Reißkräfte) liegen in Längsrichtung bei ca. 47 N/cm und in Querrichtung (quer zur Wirkrichtung) bei ca. 28 N/cm. Die Werte für die Dehnung in Längsrichtung liegen bei ca. (135 ± 20) %. In Querrichtung liegen die Werte für die Dehnung bei ca. (225 ± 3) %. Grundsätzlich kommen für den mittigen Netzabschnitt aber auch andere Netzmaterialien als Polypropylen in Betracht, wobei die Netzmaterialien vorzugsweise die gleiche Netzstruktur besitzen wie das Netz Optilene^{®} Mesh LP.

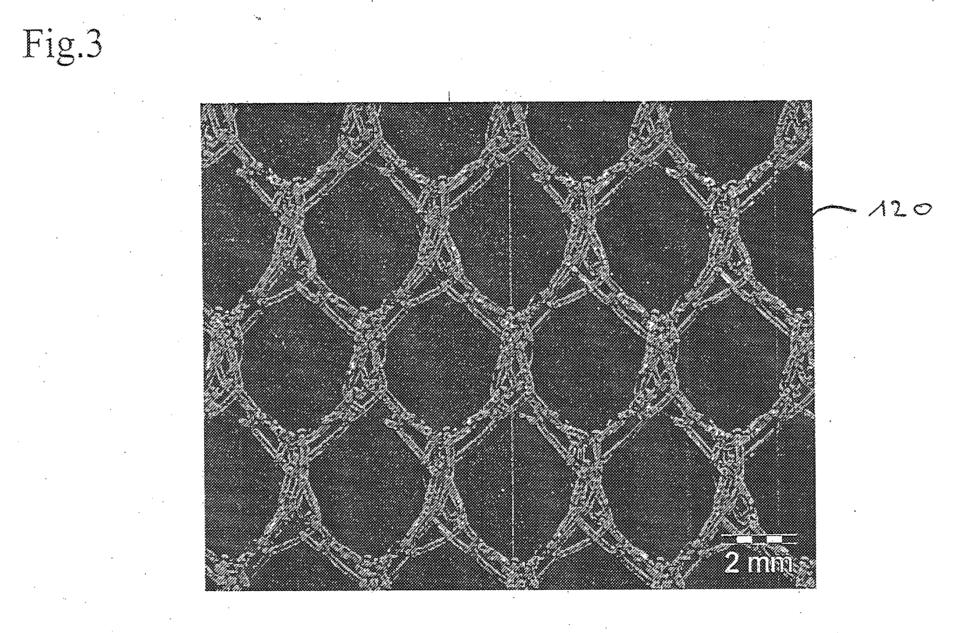

Die Figur 3 zeigt eine mikroskopische Aufnahme eines seitlichen Netzabschnittes 120, der aus dem Netz Optilene^{®} Mesh Elastic gebildet ist. Die Netzstruktur besitzt eine horizontal wie vertikal egalisierte Wabenstruktur mit verhältnismäßig großen Maschen (Maschengröße: 3,6 x 2,8 mm). Die eher offenmaschige und hexagonale Struktur der seitlichen Netzabschnitte 120 führt zu einer Maschendichte von ca. 60 Maschen pro cm². Die Netzstruktur ist ebenfalls aus monofilen Polypropylengarnen gebildet. Das Flächengewicht der Netzstruktur liegt bei ca. 48 ± 7 g/m². Die Festigkeit (Reißkraft) der Netzstruktur liegt im Mittel bei ca. 60 N/cm in Längsrichtung und im Mittel bei ca. 40 N/cm in Querrichtung der Netzstruktur. Die mittleren Dehnungswerte unterscheiden sich in Längs- und Querrichtung nur geringfügig und können mit ca. (135 ± 20) % im Wesentlichen als isotrop charakterisiert werden. Bevorzugt besitzt zumindest einer der beiden seitlichen Netzabschnitte, insbesondere beide seitlichen Netzabschnitte, die gleiche Netzstruktur wie das Netz Optilene^{®} Mesh Elastic, wobei der zumindest eine seitliche Netzabschnitt, insbesondere die beiden seitlichen Netzabschnitte, aus einem anderen Material als Polypropylen, vorzugsweise aus einem resorbierbaren Material, gebildet sind.

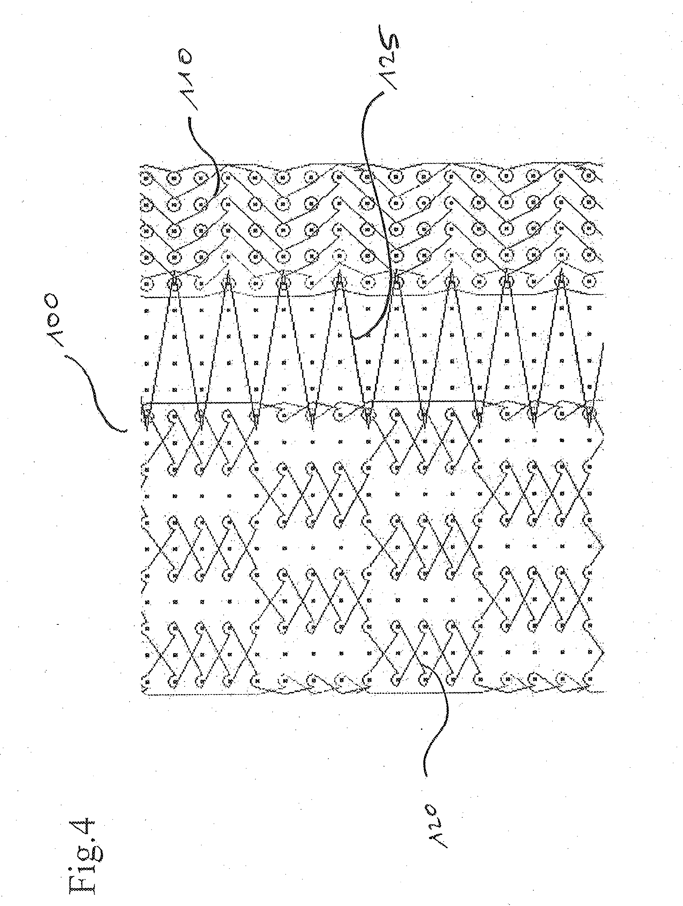

Die Figur 4 zeigt schematisch einen Ausschnitt aus einem textilen Legungsbild für ein erfindungsgemäßes Netz 100. Gezeigt ist die Verbindung zwischen dem mittigen Netzabschnitt 110 und einem seitlichen Netzabschnitt 120. Der mittige Netzabschnitt 110 ist dabei vorzugsweise aus einem Netz mit der Bezeichnung Optilene^{®} Mesh LP, welches in der vorliegenden Beschreibung näher beschrieben ist, gebildet. Der seitliche Netzabschnitt 120 ist bevorzugt aus einem Netz mit der Bezeichnung Optilene^{®} Mesh Elastic, welches ebenso in der vorliegenden Beschreibung näher erläutert ist, gebildet. Die Verbindung zwischen dem mittigen Netzabschnitt 110 und dem seitlichen Netzabschnitt 120 kommt vor allem durch den Teilschussfaden 125 zustande.

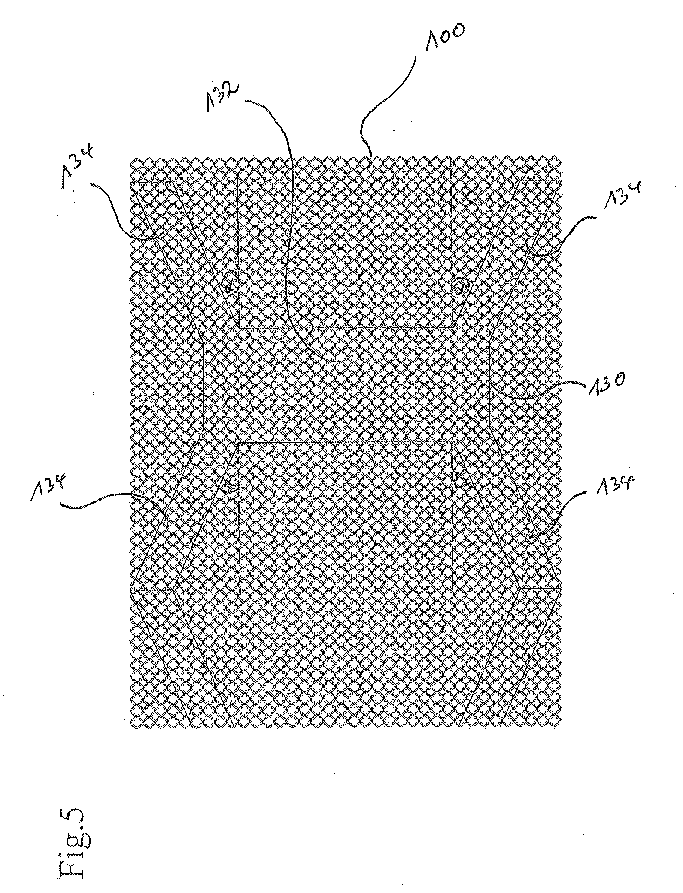

In der Figur 5 ist schematisch eine Ausführungsform für das erfindungsgemäße Netz 100 sowie die Umrisslinie eines erfindungsgemäßen chirurgischen Implantat 130 dargestellt. Das Netz 100 ist vorzugsweise aus dem Netztyp Optilene^{®} Mesh LP hergestellt. Das chirurgische Implantat 130 kann einen im Wesentlichen rechteck - oder trapezförmigen Grundkörper 132 sowie vier an den Eckbereichen des Grundkörpers 132 im Wesentlichen streifenförmig ausgebildete Fortsätze 134 aufweisen. Das chirurgische Implantat 130 wird vorzugsweise aus dem Netz 100 bzw. einer Netzbahn davon herausgeschnitten. Dies geschieht bevorzugt mittels eines Lasers. Zweckmäßigerweise erfolgt das Herausschneiden des chirurgischen Implantats 130 aus dem Netz 100 im Rahmen eines automatisierten Prozesses. Die Fortsätze 134 sind vorzugsweise um einen Winkel αvon ca. 30 Grad gegenüber der Querrichtung des Grundkörpers 132 geneigt. Die Fortsätze 134 weisen bevorzugt im Vergleich zum Grundkörper 132 eine deutlich geringere Elastizität auf, insbesondere im Vergleich zu der Elastizität des Grundkörpers 132 in dessen Querrichtung. Die Querrichtung des Grundkörpers 132 stimmt dabei mit der Längsrichtung des Netzes 100 bzw. einer Netzbahn davon überein. Der Grundkörper 132 dient vorzugsweise der flächigen Unterstützung von hervorgetretenen Organen bzw. Organteilen, wohingegen die Fortsätze 134 in der Regel zur Aufhängung des chirurgischen Implantats 130 in einer Körperdefektzone, insbesondere Prolapszone, verwendet werden.

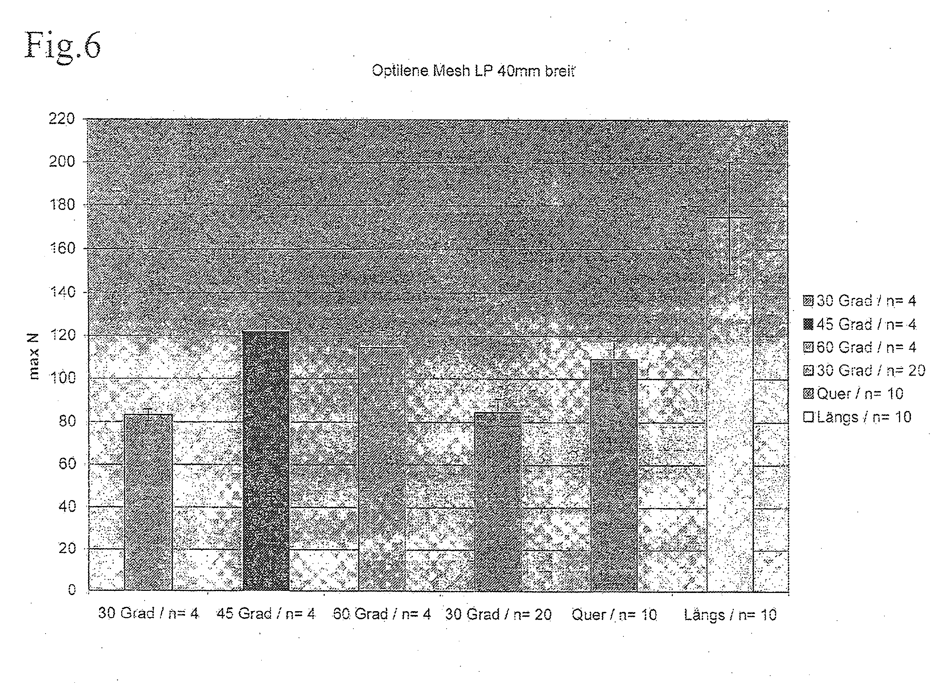

Die in der Figur 6 graphisch dargestellten Ergebnisse einer Dehnbarkeitsmessung beziehen sich auf ein erfindungsgemäßes Implantat, welches einen Grundkörper und vier seitlich vom Grundkörper abstehende Fortsätze aufweist (vgl. hierzu auch das in Figur 5 dargestellte chirurgische Implantat). Konkret wird in der Figur 6 die Dehnbarkeit der Fortsätze in Abhängigkeit von ihrem Winkel α gegenüber der Querrichtung des Grundkörpers vergleichend der Dehnbarkeit des Grundkörpers in Längs- und Querrichtung gegenübergestellt. Die in Figur 6 dargestellten Balken zeigen die Dehnung der Fortsätze bzw. des Grundkörpers bei vorgegebenen, maximalen Zugkräften in Newton [N]. Das untersuchte Implantat wurde dabei aus einer Netzbahn von Optilene^{®} Mesh LP herausgeschnitten. Die in Figur 6 dargestellten Messergebnisse machen deutlich, dass Fortsätze mit einem Winkel α von 30, 45 oder 60 Grad gegenüber der Querrichtung des Grundkörpers eine signifikant geringere Dehnbarkeit in Längsrichtung aufweisen als der Grundkörper in Längsrichtung. Insbesondere weisen Fortsätze in Längsrichtung, welche gegenüber der Querrichtung des Grundkörpers um einen Winkel von 30 Grad geneigt sind, sogar eine deutlich geringere Dehnbarkeit auf als der Grundkörper in dessen Querrichtung.

### Beispiel: Aufbau eines Gradienten-Netzes

### Netzanteil aus Optilene^{®} Mesh LP:

Garntiter: 90 dtex
Garndurchmesser: 0,1 mm
Bindung: 2-reihiger Atlas geschlossen
Kettaufstellung (1-0/2-1/2-3/1-2) x 2 für Legeschiene 2

### Netzanteil aus Optilene^{®} Mesh Elastic:

Garntiter: 150 dtex
Garndurchmesser: 0,145 mm
Bindung: Filet offen
Kettaufstellung: 0-1/3-2/0-1/2-3/5-4/2-3/5-4/3-2 für Legeschiene 3
   5-4/2-3/5-4/3-2/0-1/3-2/0-1/2-3 für Legeschiene 4

### Teilschuss für Verbindung der beiden obigen Netzanteile:

Garntiter: 150 dtex
Garndurchmesser: 0,145 mm
Bindung: Teilschuss
Kettaufstellung: 5-5/6-6/5-5/6-6/0-0/6-6/0-0/6-6 für Legeschiene 5
   1-1/0-0/1-1/0-0/6-6/0-0/6-6/0-0 für Legeschiene 6

## Patentansprüche

1. Textiles Netz (100), insbesondere zur Beckenbodehrekonstruktion oder zur Vermeidung von Dyspareunia, umfassend einen mittigen Netzabschnitt (110) und zwei seitliche Netzabschnitte (120) beidseits des mittigen Netzabschnittes (110), wobei das Netz (100) zumindest teilweise anisotrop elastisch ausgebildet ist, das Netz (100) im Bereich des mittigen Netzabschnittes (110) elastischer ausgebildet ist als in den Bereichen der beiden seitlichen Netzabschnitte (120), und der mittige Netzabschnitt (110) nicht resorbierbar und die beiden seitlichen Netzabschnitte (120) nicht resorbierbar ausgebildet sind, **dadurch gekennzeichnet, dass** die beiden seitlichen Netzabschnitte (120) in einer Trikot-Atlasbindung ausgeführt sind.

2. Textiles Netz (100) nach Anspruch 1, **dadurch gekennzeichnet, dass** das Netz (100) in Querrichtung des mittigen Netzabschnittes (110) elastischer ausgebildet ist als in Längsrichtung davon.

3. Textiles Netz (100) nach Anspruch 1 oder 2, **dadurch gekennzeichnet, dass** das Netz (100) in Querrichtung des mittigen Netzabschnittes (110) elastischer ausgebildet ist als in Längs- und/oder Querrichtung, vorzugsweise Längs- und Querrichtung, der beiden seitlichen Netzabschnitte (120).

4. Textiles Netz (100) nach einem der vorhergehenden Ansprüche, **dadurch gekennzeichnet, dass** das Netz (100) als Gradientennetz ausgebildet ist, vorzugsweise der mittige Netzabschnitt (110) eine andere textile Netzstruktur besitzt als die beiden seitlichen Netzabschnitte (120).

5. Textiles Netz (100) nach einem der vorhergehenden Ansprüche, **dadurch gekennzeichnet, dass** Fäden des mittigen Netzabschnittes (110) einen anderen Durchmesser besitzen als Fäden der beiden seitlichen Netzabschnitte (120).

6. Textiles Netz (100) nach einem der vorhergehenden Ansprüche, **dadurch gekennzeichnet, dass** der mittige Netzabschnitt (110) eine andere Maschenstruktur besitzt als die beiden seitlichen Netzabschnitte (120).

7. Textiles Netz (100) nach einem der vorhergehenden Ansprüche, **dadurch gekennzeichnet, dass** der mittige Netzabschnitt (110) ein leichteres Flächengewicht als die beiden seitlichen Netzabschnitte (120) besitzt.

8. Textiles Netz (100) nach einem der vorhergehenden Ansprüche, **dadurch gekennzeichnet, dass** der mittige Netzabschnitt (110) eine gewirkte und vorzugsweise veloursfreie Netzstruktur und die beiden seitlichen Netzabschnitte (120) eine gewirkte Veloursstruktur, insbesondere eine gewirkte Doppelveloursstruktur, besitzen.

9. Textiles Netz (100) nach einem der Ansprüche 1 bis 7, **dadurch gekennzeichnet, dass** der mittige Netzabschnitt (110) eine gewirkte und vorzugsweise veloursfreie Netzstruktur und die beiden seitlichen Netzabschnitte (120) eine dreidimensionale Textilstruktur, insbesondere eine dreidimensionale Abstandstextilstruktur, besitzen.

10. Textiles Netz (100) nach einem der vorhergehenden Ansprüche, **dadurch gekennzeichnet, dass** das Netz (100), einstückig gewirkt ausgebildet, in, Trikot-Atlas-bindung ausgeführt ist.

11. Textiles Netz (100) nach einem der vorhergehenden Ansprüche, **dadurch gekennzeichnet, dass** der mittige Netzabschnitt (110) Polyolefinfäden, vorzugsweise Polypropylenfäden, aufweist bzw. aus solchen Fäden gebildet ist.

12. Textiles Netz (100) nach einem der vorhergehenden Ansprüche, **dadurch gekennzeichnet, dass** der mittige Netzabschnitt (110) antimikrobielle, vorzugsweise antibakterielle, Eigenschaften besitzt.

13. Chirurgisches Implantat (130), insbesondere zur Beckenbodenrekonstruktion oder zur Vermeidung von Dyspareunia, hergestellt bzw. gefertigt aus einem textilen Netz (100) nach einem der Ansprüche 1 bis 12, wobei das Implantat (130) einen Grundkörper (132) und vorzugsweise streifenförmige Fortsätze (134) aufweist, wobei vorzugsweise der Grundkörper (132) aus dem mittigen Netzabschnitt (110) und die Fortsätze (134) aus den seitlichen Netzabschnitten (120) des Netzes (100) gebildet sind.

14. Chirurgisches Implantat (130) nach Anspruch 13, **dadurch gekennzeichnet, dass** der Grundkörper (132) eine gewirkte und vorzugsweise veloursfreie Netzstruktur und die Fortsätze (134) eine gewirkte Veloursstruktur, insbesondere eine gewirkte Doppelveloursstruktur, besitzen.

15. Chirurgisches Implantat (130) nach Anspruch 13 oder 14, **dadurch gekennzeichnet, dass** der Grundkörper (132) eine gewirkte und vorzugsweise veloursfreie Netzstruktur und die Fortsätze (134) eine dreidimensionale Textilstruktur, insbesondere eine dreidimensionale Abstandstextilstruktur, besitzen.

## Claims

1. Textile mesh (100), in particular for pelvic floor reconstruction or for preventing dyspareunia, comprising a central mesh section (110) and two lateral mesh sections (120) on both sides of the central mesh section (110), wherein the mesh (100) at least partially has anisotropic elasticity, the mesh (100) being more elastic in the region of the central mesh section (110) than in the regions of the two lateral mesh sections (120), and the central mesh section (110) is not absorbable and the two lateral mesh sections (120) are not absorbable,
**characterized in that**
the two lateral mesh sections (120) are constructed in a tricot satin weave.

2. Textile mesh (100) according to claim 1, **characterized in that** the mesh (100) is more elastic in the transverse direction of the central mesh section (110) than in the longitudinal direction thereof.

3. Textile mesh (100) according to claim 1 or 2, **characterized in that** the mesh (100) is more elastic in the transverse direction of the central mesh section (110) than in the longitudinal and/or transverse direction, preferably longitudinal and transverse direction, of the two lateral mesh sections (120).

4. Textile mesh (100) according to any one of the preceding claims, **characterized in that** the mesh (100) is a gradient mesh, preferably the central mesh section (110) has a textile mesh structure differing from that of the two lateral mesh sections (120).

5. Textile mesh (100) according to any one of the preceding claims, **characterized in that** the threads of the central mesh section (110) have a diameter differing from that of threads in the two lateral mesh sections (120).

6. Textile mesh (100) according to any one of the preceding claims, **characterized in that** the central mesh section (110) has a stitch structure differing from that of the two lateral mesh sections (120).

7. Textile mesh (100) according to any one of the preceding claims, **characterized in that** the central mesh section (110) has a smaller mass per unit area than the two lateral mesh sections (120).

8. Textile mesh (100) according to any one of the preceding claims, **characterized in that** the central mesh section (110) has a knitted and preferably velours-free mesh structure and the two lateral mesh sections (120) have a knitted velours structure, in particular a knitted double-velours structure.

9. Textile mesh (100) according to any one of claims 1 to 7, **characterized in that** the central mesh section (110) has a knitted and preferably velours-free mesh structure and the two lateral mesh sections (120) have a three-dimensional textile structure, in particular a three-dimensional spacer textile structure.

10. Textile mesh (100) according to any one of the preceding claims, **characterized in that** the mesh (100) is, knitted in one piece, constructed in a tricot satin weave.

11. Textile mesh (100) according to any one of the preceding claims, **characterized in that** the central mesh section (110) includes polyolefin fibres, preferably polypropylene fibres, or is made of such fibres.

12. Textile mesh (100) according to any one of the preceding claims, **characterized in that** the central mesh section (110) has antimicrobial, preferably antibacterial, characteristics.

13. Surgical implant (130), in particular for pelvic floor reconstruction or for preventing dyspareunia, produced and/or manufactured from a textile mesh (100) according to any one of claims 1 to 12, wherein the implant (130) has a base body (132) and preferably strip-type extensions (134), wherein preferably the base body (132) is composed of the central mesh section (110) and the extensions (134) are composed of the lateral mesh sections (120) of the mesh (100).

14. Surgical implant (130) according to claim 13, **characterized in that** the base body (132) has a knitted and preferably velours-free mesh structure and the extensions (134) have a knitted velours structure, in particular a knitted double-velours structure.

15. Surgical implant (130) according to claim 13 or 14, **characterized in that** the base body (132) has a knitted and preferably velours-free mesh structure and the extensions (134) have a three-dimensional textile structure, in particular a three-dimensional spacer textile structure.

## Revendications

1. Filet textile (100), en particulier pour la reconstruction du plancher pelvien ou pour éviter une dyspareunie, comprenant une portion de filet centrale (110) et deux portions de filet latérales (120) de part et d'autre de la portion de filet centrale (110), dans lequel le filet (100) est au moins en partie élastiquement anisotrope, le filet (100) est plus élastique dans la région de la portion de filet centrale (110) que dans les régions des deux portions de filet latérales (120), et la portion de filet centrale (110) n'est pas résorbable et les deux portions de filet latérales (120) ne sont pas résorbables, **caractérisé en ce que** les deux portions de filet latérales (120) sont réalisées en une armure atlas tricot.

2. Filet textile (100) selon la revendication 1, **caractérisé en ce que** le filet (100) est plus élastique en direction transversale de la portion de filet centrale (110) qu'en direction longitudinale de celle-ci.

3. Filet textile (100) selon la revendication 1 ou 2, **caractérisé en ce que** le filet (100) est plus élastique en direction transversale de la portion de filet centrale (110) qu'en direction longitudinale et/ou transversale, de préférence en direction longitudinale et transversale, des deux portions de filet latérales (120).

4. Filet textile (100) selon l'une quelconque des revendications précédentes, **caractérisé en ce que** le filet (100) est réalisé sous forme de filet en gradient, de préférence la portion de filet centrale (110) présente une autre structure de filet textile que les deux portions de filet latérales (120).

5. Filet textile (100) selon l'une quelconque des revendications précédentes, **caractérisé en ce que** des fils de la portion de filet centrale (110) présentent un autre diamètre que des fils des deux portions de filet latérales (120).

6. Filet textile (100) selon l'une quelconque des revendications précédentes, **caractérisé en ce que** la portion de filet centrale (110) présente une autre structure de mailles que les deux portions de filet latérales (120).

7. Filet textile (100) selon l'une quelconque des revendications précédentes, **caractérisé en ce que** la portion de filet centrale (110) présente un poids par unité de surface plus faible que les deux portions de filet latérales (120).

8. Filet textile (100) selon l'une quelconque des revendications précédentes, **caractérisé en ce que** la portion de filet centrale (110) présente une structure de filet tricotée et de préférence sans velours et les deux portions de filet latérales (120) présentent une structure tricotée velours, en particulier une structure tricotée double velours.

9. Filet textile (100) selon l'une quelconque des revendications 1 à 7, **caractérisé en ce que** la portion de filet centrale (110) présente une structure de filet tricotée et de préférence sans velours et les deux portions de filet latérales (120) présentent une structure textile tridimensionnelle, en particulier une structure textile tridimensionnelle écartée.

10. Filet textile (100) selon l'une quelconque des revendications précédentes, **caractérisé en ce que** le filet (100), tricoté d'une seule pièce, est réalisé en armure atlas tricot.

11. Filet textile (100) selon l'une quelconque des revendications précédentes, **caractérisé en ce que** la portion de filet centrale (110) présente des fils de polyoléfine, de préférence des fils de polypropylène, ou est formée de tels fils.

12. Filet textile (100) selon l'une quelconque des revendications précédentes, **caractérisé en ce que** la portion de filet centrale (110) possède des propriétés antimicrobiennes, de préférence antibactériennes.

13. Implant chirurgical (130), en particulier pour la reconstruction du plancher pelvien ou pour éviter une dyspareunie, produit ou fabriqué à partir d'un filet textile (100) selon l'une quelconque des revendications 1 à 12, dans lequel l'implant (130) présente un corps de base (132) et des prolongements (134) de préférence en forme de bandes, de préférence dans lequel le corps de base (132) est formé de la portion de filet centrale (110) et les prolongements (134) sont formés des portions de filet latérales (120) du filet (100).

14. Implant chirurgical (130) selon la revendication 13, **caractérisé en ce que** le corps de base (132) présente une structure de filet tricotée et de préférence sans velours et les prolongements (134) présentent une structure tricotée velours, en particulier une structure tricotée double velours.

15. Implant chirurgical (130) selon la revendication 13 ou 14, **caractérisé en ce que** le corps de base (132) présente une structure de filet tricotée et de préférence sans velours et les prolongements (134) présentent une structure textile tridimensionnelle, en particulier une structure textile tridimensionnelle écartée.
